# FASCICULE DE BREVET EUROPEEN

(11) **EP 2 188 253 B1**
(45) Date de publication et mention de la délivrance du brevet: **07.03.2012**
(21) Numéro de dépôt: 08845678.5
(22) Date de dépôt: 14.08.2008
(51) Int. Cl.: C07D 209/40, C07D 401/12, A61K 31/454, A61K 31/496, A61P 3/00, C07D 401/14

(54) **Dérivés de l'indol-2-one disubstitués en 3, leur préparation et leur application en thérapeutique**
In Stellung 3 disubstituierte Indol-2-on-pyridinderivate, ihre Herstellung sowie ihre therapeutische Anwendung
Indol-2-one derivatives disubstituted in 3-position, preparation thereof and therapeutic use thereof

(30) Priorité: 16.08.2007 FR 0705858
(43) Date de publication de la demande: 26.05.2010
(73) Titulaire: SANOFI, 75013 Paris (FR); Puleo, Letizia, 75013 Paris (FR)
(72) Inventeur: BARONI, Marco, 75013 Paris (FR); PULEO, Letizia, F-75013 Paris (FR)
(74) Mandataire: Rauline, Mathilde
(86) Numéro de dépôt international: PCT/FR2008/001190
(87) Numéro de publication internationale: WO 2009/056707

(56) Documents cités:
- WO-A-00/10975
- WO-A-95/18105
- WO-A-03/008407
- WO-A-2005/035498

## Description

La présente invention a pour objet des dérivés de l'indol-2-one disubstitués en 3, leur préparation et leur application en thérapeutique.

La ghréline est une hormone peptidique de 28 acides aminés produite de façon principale dans l'estomac par un processus post-traductionnel après clivage de la pré-pro-ghréline (Kojima M, et al., Nature 1999; 402:656-60). La ghréline est un ligand endogène du récepteur hypophysaire des sécrétagogues de l'hormone de croissance (GHSR1a).

GHS-R est codé par deux exons : l'exon 1 code pour les domaines transmembranaires (TMs) 1-5 et l'exon 2 code pour TM6 et 7 du récepteur couplé à la protéine G (GPCR).

Les deux transcripts ont été identifiés dans la glande pituitaire et le cerveau: l'un codant pour le GPCR de longueur totale (GHS-R1a) et l'autre codant pour un récepteur tronqué (GHS-R1b) auquel manquent TM6 et 7. Seul le sous-type GHS-R1a est activé par la ghréline et les mimétiques de la ghréline. GHS-R1b est présent dans le foie et d'autres tissus périphériques mais sa fonction est inconnue (Smith R.G. et al, Trends in Endocrinology and Métabolism, 2005, 16 No. 9).

C'est un récepteur de type rhodopsine, à sept domaines transmembranaires de la famille A couplé à Gq/phospholipase C. Le récepteur de la ghréline peut aussi être couplé aux voies Gs/protéine kinase A dans certains tissus (Ueno, N. et al. Endocrinology, 2004, 145, 4176-4184 ; Kim, M.S. et al., Int. J. Obes. Relat. Metab. Disord., 2004, 28:1264-1271). De façon intéressante, le récepteur de la ghréline présente la caractéristique peu commune de présenter une activité constitutive indépendante du ligand significative (Barazzoni, R. et al., Am. J. Physiol. Endocrinol. Metab., 2004, 288: E228-E235).

Des niveaux faibles de l'expression de la ghréline ont été documentés dans des tissus variés, tels que les intestins, le pancréas, les reins, le système immunitaire, le placenta, les testicules, les tissus pituitaires, l'hypothalamus (Horm. Res. 2003; 59 (3):109-17).

Il a été démontré que la ghréline est impliquée dans la faim au moment des repas, et dans l'initiation des repas. Les niveaux circulants diminuent avec la prise d'alimentation et augmentent avant les repas, atteignant des concentrations suffisantes pour stimuler la faim et la prise de nourriture. Les ingestions de ghréline stimulent la prise de nourriture rapidement et de façon transitoire, principalement en augmentant les comportements d'alimentation appétante et le nombre de repas. La ghréline stimule la prise de nourriture à court terme de façon plus efficace que n'importe quelle autre molécule, à l'exception du neuropeptide Y, avec lequel elle est approximativement équipotente (Wren A.M., et al., J. Clin. Endocrinol. Metab., 2001;86:5992-5.). Cependant, la ghréline est unique dans sa capacité à exercer cet effet, qu'elle soit injectée de façon périphérique ou centrale.
C'est également la seule substance de mammifère qui a démontré sa capacité à augmenter l'appétit et la prise d'alimentation lorsqu'elle est administrée aux humains (Druce M.R., et al., Int. J. Obes., 2005; 29:1130-6; Wynne K., et al., J. Am. Soc. Nephrol., 2005; 16: 2111-8.).

Au-delà de son rôle dans l'initiation des repas, la ghréline remplit également les critères établis d'une hormone liée à l'adiposité impliquée dans la régulation de la masse corporelle à long terme. Les taux de ghréline circulent en fonction des stocks énergétiques et manifestent des changements compensatoires en réponse aux modifications de la masse corporelle.

La ghréline traverse la barrière hémato-encéphalique et stimule la prise de nourriture en agissant sur certains centres régulateurs classiques de la masse corporelle, tels que l'hypothalamus, le cerveau postérieur et le système compensatoire mésolimbique.

L'administration chronique de ghréline augmente la masse corporelle par diverses actions concertées sur la prise de nourriture, la dépense énergétique et l'utilisation des ressources. L'ablation congénitale de la ghréline ou du gène du récepteur de la ghréline provoque une résistance à l'obésité induite par l'alimentation, et le blocage pharmacologique de la ghréline réduit la prise de nourriture et la masse corporelle.

Les preuves existantes semblent favoriser le rôle de la ghréline à la fois dans l'initiation des repas à court terme et l'homéostasie énergétique à long terme, en faisant ainsi une cible attractive comme médicament pour traiter l'obésité et/ou les désordres de l'amaigrissement.
La ghréline exerce également des actions à la fois physiologiques et pharmacologiques sur le pancréas endocrine. La ghréline bioactive acylée est produite dans la cellule-ε, récemment décrite dans les îlots pancréatiques (Prado, C.L., et al., 2004, Proc. Natl. Acad. Sci. USA, 101:2924-2929), fournissant potentiellement une source locale de ghréline qui agit sur les cellules β des îlots. Le blocage de cette fonction de la ghréline endogène par le biais d'un antagoniste pour ses récepteurs a diminué les concentrations de glucose à jeûn de façon prononcée, a atténué le mouvement glycémique et augmenté les réponses à l'insuline lors des tests à la tolérance au glucose, suggérant un rôle inhibiteur pour la ghréline dans le contrôle de la sécrétion de l'insuline (Dezaki, K., et al. 2004, Diabètes, 53:3142-3151).
L'ablation de la ghréline chez les souris (souris ghréline -/-) augmente la sécrétion d'insuline dépendante du glucose par la cellule β du pancréas par réduction de l'expression Ucp2 et améliore la sensibilité à l'insuline périphérique (Sun Y. et al., 2006, Cell Metabolism, 3:379-386).

Des antagonistes du récepteur de la ghréline pourraient ainsi réguler la faim, la prise de repas et leur fréquence, ainsi que, à long terme, le poids, notamment la prise de poids faisant suite aux régimes diététiques ou thérapeutiques. De plus, dans le cadre d'un traitement antidiabétique, les antagonistes de la ghréline pourraient être utiles pour maintenir l'équilibre entre insuline et le glucose et pour contrôler l'hyperphagie diabétique. Les antagonistes de la ghréline pourraient ainsi être utilisés comme agents anorexiques et/ou anti-obésité, ou encore dans le traitement du diabète et de ses effets.

La demande de brevet WO 95/18105 décrit les composés 5-chloro-3-(2-chlorophényl)-1,3-dihydro-3-[2-(4-méthylpiperazin-1-yl)acétamido]indol-2-one et 5-chloro-3-(2-chloroacétamido)-3-(2-chlorophényl)-1,3-dihydroindol-2-one en tant qu'intermédiaires de synthèse de dérivés de 1, 3- dihydroindol-2-one substitués en -3- par un groupe azoté et ayant une affinité pour la vasopressine et/ou l'ocytocine.

La présente invention a pour objet les composés répondant à la formule (I) : 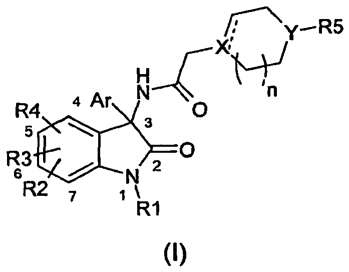 dans laquelle : 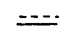
représente une simple ou double liaison,
X représente -N<, -CH< ou 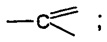
Y représente >N- ou >CH-, étant entendu qu'au moins un des X, Y représente N ;
Ar représente un groupe aryle ou hétéroaryle éventuellement substitué par un plusieurs substituants, identiques ou différents, choisis parmi les atomes d'halogène, les groupes (C1-6) alkyle, (C1-6) halogénoalkyle, perhalogéno(C1-3) alkyle, (C1-6) alcoxy, perhalogéno(C1-3) alcoxy, aryle;
R1 représente un atome d'hydrogène ou un groupe (C1-6) alkyle, - C(=O)(C₁₋₆) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle éventuellement substitué par un atome d'halogène ou un OH ; perhalogéno(C1-3) alkyle, (C1-6) alcoxy, perhalogéno(C1-3) alcoxy, aminocarbonyle, (C1-6) alkylaminocarbonyle, di(C1-6) alkylaminocarbonyle, aryle, aryloxy ; hétéroaryle ; le groupe aryle, aryloxy ou hétéroaryle pouvant éventuellement être substitué par un atome d'halogène, CN, OH ou un groupe (C1-6) alkyle , perhalogéno(C1-3) alkyle ou (C1-6) alcoxy ; étant entendu que au moins un des R2, R3, R4 est différent de H et que le groupe aryle, aryloxy ou hétéroaryle peut éventuellement être substitué par un atome d'halogène, CN, OH ou un groupe (C1-6) alkyle , perhalogéno(C1-3) alkyle, (C1-6) alcoxy;
R5 représente un groupe (C1-6) alkyle ou un (C2-6) alcényle;et
n représente 1 ou 2 ; à l'exclusion du 5-chloro-3-(2-chlorophényl)-1,3-dihydro-3-[2-(4-méthylpiperazin-1-yl)acétamido]indol-2-one.

Les composés de formule (I) comportent un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Dans le cadre de la présente invention, on entend par :
- un atome d'halogène : un fluor, un chlore, un brome ou un iode ;
- un groupe alkyle : un groupe aliphatique saturé linéaire ou ramifié. A titre d'exemples, on peut citer un groupe (C1-6) alkyle comprenant de 1 à 6 atomes de carbone, plus particulièrement (C1-4) alkyle qui peut représenter un méthyle, éthyle, propyle, isopropyle, butyle, isobutyle, tertbutyle ;
- un groupe alcényle : un groupe aliphatique mono- ou poly-insaturé, linéaire ou ramifié, comprenant par exemple une ou deux insaturations et comprenant de 2 à 6 atomes de carbone ;
- un groupe halogénoalkyle un groupe alkyle dont un ou plusieurs atomes d'hydrogène ont été substitués par un atome d'halogène ; par exemple un fluoroalkyle: un groupe alkyle dont un ou plusieurs atomes d'hydrogène ont été substitués par un atome de fluor ;
- un groupe perhalogénoalklyle : un groupe alkyle dont tous les atomes d'hydrogène ont été substitués par un atome d'halogène ; par exemple un perfluoroalkyle : un groupe alkyle dont tous les atomes d'hydrogène ont été substitués par un atome de fluor ;
- un groupe alcoxy : un radical -O-alkyle où le groupe alkyle est tel que précédemment défini;
- un groupe perhalogénoalcoxy : un radical -O-perhalogénoalkyle où le groupe perhalogénoalkyle est tel que précédemment défini, à titre d'exemple on peut citer le trifluorométhoxy;
- un groupe aryle : un groupe aromatique cyclique comprenant entre 6 et 10 atomes de carbone. A titre d'exemples de groupes aryles, on peut citer un phényle ou naphthyle;
- un groupe hétéroaryle : un groupe aromatique cyclique comprenant entre 2 et 10 atomes de carbone et comprenant entre 1 et 3 hétéroatomes, tels que l'azote, l'oxygène ou le soufre. A titre d'exemples de groupes hétéroaryles, on peut citer les groupes furanyle, pyrrolyle, imidazolyle, pyrazolyle, thiényle, oxadiazolyle, oxazolyle, isoxazolyle, furazanyle, thiadiazolyle, thiazolyle, isothiazolyle, pyridyle, pyrazinyle, pyrimidinyle, pyridazinyle ainsi que les groupes correspondants résultants de la fusion avec un groupe phényle tel que par exemple benzothiophène, benzofurane; benzothiazole, ...

Parmi les composés de formule (I) objets de l'invention, un groupe de composés est constitué par les composés pour lesquels :
Ar représente un groupe aryle ou hétéroaryle éventuellement substitué par un ou plusieurs substituants, identiques ou différents, choisis parmi les atomes d'halogène, les groupes (C1-6) alkyle, perhalogéno(C1-3) alkyle, (C1-6) alcoxy, aryle;
R1 représente un atome d'hydrogène ou un groupe (C1-6) alkyle, - C(=O)(C1-6) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN, OH, un groupe (C1-6) alkyle, perhalogéno(C1-3) alkyle, (C1-6) alcoxy, perhalogéno(C1-3) alcoxy , aminocarbonyle, (C1-6) alkylaminocarbonyle, di(C1-6) alkylaminocarbonyle, aryle, aryloxy ; hétéroaryle, étant entendu que au moins un des R2, R3, R4 est différent de H ;
R5 représente un groupe (C1-6) alkyle ;

Parmi les composés de formule (I) objets de l'invention, un groupe de composés est constitué par les composés pour lesquels : 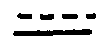
représente une simple ou double liaison, et/ou
X représente -N<, -CH< ou 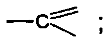 et/ou
Y représente >N- ou >CH- ; et ou
étant entendu qu'au moins un des X, Y représente N ; et/ou
Ar représente un groupe aryle éventuellement substitué par un ou plusieurs substituants choisis parmi les atomes d'halogène, préférentiellement le chlore ou le brome, et les groupes (C1-6) alcoxy, (C1-6) alkyle, aryle, trifluorométhyle, trifluorométhoxy ; et/ou
R1 représente un atome d'hydrogène ou un groupe -C(=O)(C1-6) alkyle, -C(=O)aryle, (C1-6) alkyle ; et/ou
R2, R3, R4, identiques ou différents, situés sur l'une quelconque des positions disponibles du noyau phényle, représentent indépendamment un atome d'hydrogène, un atome d'halogène, préférentiellement le chlore ou le brome, ou un groupe (C1-6) alkyle ou trifluorométhyle, étant entendu que au moins un des R2, R3, R4 est différent de H ; et/ou
R5 représente un groupe (C1-6) alkyle ; et/ou
n représente 1 ou 2 ;
sous forme de base ou de sel d'addition à un acide.

Parmi les composés de formule (I) objets de l'invention, un autre groupe de composés est constitué par les composés pour lesquels : 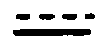
représente une simple ou double liaison, et/ou
X représente -N<, -CH< ou 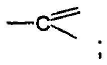 et/ou
Y représente >N- ou >CH- ; et ou
étant entendu qu'au moins un des X, Y représente N ; et/ou
Ar représente un groupe phényle ou naphtyle éventuellement substitué par un ou plusieurs substituants choisis parmi les atomes d'halogène, préférentiellement le chlore ou le brome, et les groupes méthoxy, méthyle, tert-butyle, phényle, trifluorométhyle, trifluorométhoxy ; et/ou
R1 représente un atome d'hydrogène ou un groupe -C(=O)méthyle, -C(=O)phényle, méthyle ; et/ou
R2, R3, R4, identiques ou différents, situés sur l'une quelconque des positions disponibles du noyau phényle, représentent indépendamment un atome d'hydrogène, un atome d'halogène, préférentiellement le chlore ou le brome, ou un groupe méthyle ou trifluorométhyle, étant entendu que au moins un des R2, R3, R4 est différent de H ; et/ou
R5 représente un groupe méthyle, éthyle ou 2-propyle; et/ou
n représente 1 ou 2 ;
sous forme de base ou de sel d'addition à un acide.

Parmi les composés de formule (I) objets de l'invention, un autre groupe de composés est constitué par les composés pour lesquels :
Ar représente un groupe hétéroaryle éventuellement substitué par un ou plusieurs substituants, identiques ou différents, choisis parmi les atomes d'halogène, les groupes (C1-6) alkyle, perhalogéno(C1-3) alkyle, (C1-6) alcoxy, aryle.

Parmi les composés de formule (I) objets de l'invention, on peut notamment citer les composés suivants :
(+) N-[5,6-Dichloro-3-(4-chloro-phényl)-2-oxo-2,3-dihydro-1H-indol-3-yl]-2-(4-méthyl-pipérazin-1-yl)-acétamide
(+) N-[4,6-Dichloro-3-(4-trifluorométhyl-phényl)-2-oxo-2,3-dihydro-1H-indol-3-yl]-2-(1-ethyl-piperidin-4-yl)-acétamide
N-[4,6-Dichloro-3-(4-chloro-phényl)-2-oxo-2,3-dihydro-1H-indol-3-yl]-2-(4-méthyl-pipérazin-1-yl)-acétamide
N-[4-trifluoromethyl-6-cyano-3-(4-chloro-phényl)-2-oxo-2,3-dihydro-1H-indol-3-yl]-2-(4-éthyl-pipérazin-1-yl)-acétamide
(+) N-[1-Benzoyl-5,6-Dichloro-3-(4-chloro-phényl)-2-oxo-2,3-dihydro-1H-indol-3-yl]-2-(4-méthyl-pipérazin-1-yl)-acétamide
3-(4-Chloro-phényl)-3-[2-(4-ethyl-pipérazin-1-yl)-acétylamino]-2-oxo-4-trifluorométhyl-2,3-dihydro-1H-indole-6-carboxamide
N-[6-Chloro-3-(4-chloro-phényl)-1,5-diméthyl-2-oxo-2,3-dihydro-1H-indol-3-yl]-2-(4-méthyl-pipérazin-1-yl)-acétamide
(+) N-[4,6-Dichloro-3-(4-trifluorométhyl-phényl)-2-oxo-2,3-dihydro-1H-indol-3-yl]-2-(1-ethyl-1,2,3,6-tetrahydro-pyrid-4-yl)-acétamide ;
N-[4,6-Dichloro-3-(3,4-dichloro-phényl)-2-oxo-2,3-dihydro-1H-indol-3-yl]-2-(4-éthyl-pipérazin-1-yl)-acétamide
N-[4,6-Dichloro-3-(3-fluoro-4-chloro-phényl)-2-oxo-2,3-dihydro-1H-indol-3-yl]-2-(4-éthyl-pipérazin-1-yl)-acétamide
N-[4,6-Dichloro-3-(3-trifluorométhyl-4-chloro-phényl)-2-oxo-2,3-dihydro-1H-indol-3-yl]-2-(4-éthyl-pipérazin-1-yl)-acétamide
N-[4,6-Dichloro-1-ethyl-3-(2-methyl-benzo[b]thiophen-5-yl)-2-oxo-2,3-dihydro-1H-indol-3-yl]-2-(1-ethyt-piperidin-4-yl)-acétamide
N-[4,6-Dichloro-1-ethyl-3-(2-methyl-benzofuran-5-yl)-2-oxo-2,3-dihydro -1 H-indol-3-yl]-2-(4-ethyl-piperazin-1-yl)-acetamide
N-[4,6-Dichloro-3-(4-trifluorométhoxy-phényl)-2-oxo-2,3-dihydro-1H-indol-3-yl]-2-(4-éthyl-pipérazin-1-yl)-acétamide
sous forme de base ou de sel d'addition à un acide.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans Protective Groups in Organic Synthesis, Green et al., 2nd Edition (John Wiley & Sons, Inc., New York).

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans Advances in Organic Chemistry, J. March, 3rd Edition, Wiley Interscience, p. 310-316.

Conformément à l'invention, on peut préparer les composés de formule générale (I) selon le procédé qui suit :

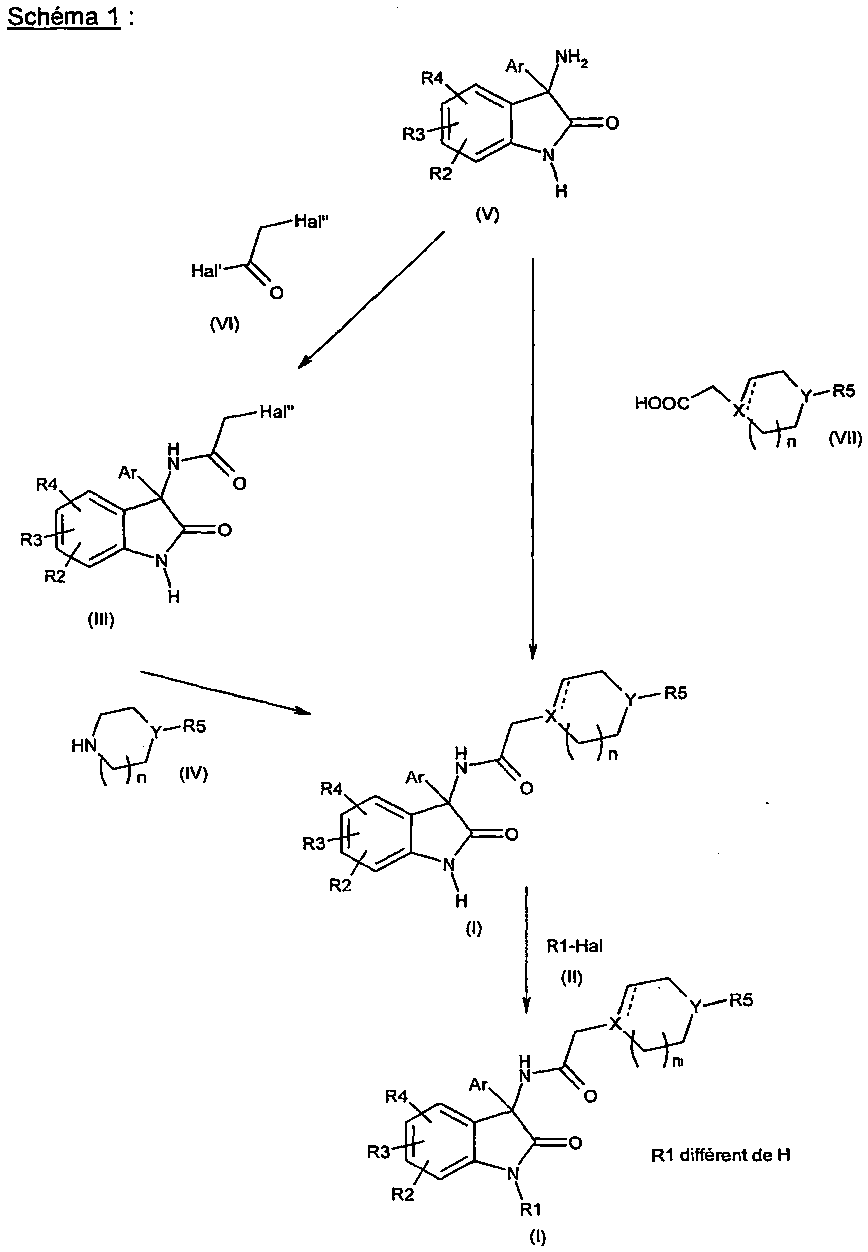

Le composé de formule (I), dans laquelle R1 est différent de H et 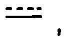 R2, R3, R4, R5, Ar, X, Y et n sont tels que définis en formule générale (I) peut être préparé par réaction d'un composé de formule (I) dans laquelle R1=H avec un composé de formule (II) :

R1-Hal (II)

dans laquelle R1, différent de H est défini comme en formule générale (I) et Hal représente un atome d'halogène, par exemple le chlore selon des méthodes connues de l'homme du métier par exemple en présence d'une base, telle que K₂CO₃, NaH, t-Bu0⁻K⁺, dans un solvant tel que le diméthylformamide (DMF), tétrahydrofurane (THF), dimethoxyéthane, le diméthylsulfoxide (DMSO).

Le composé de formule générale (I) dans laquelle R1=H peut être préparé selon l'une ou l'autres des variantes suivantes :

Lorsque X=-N<, le composé de formule générale (I) dans laquelle R1=H, c'est-à-dire le composé de formule générale (II) peut être préparé par réaction d'un composé de formule générale (III) : 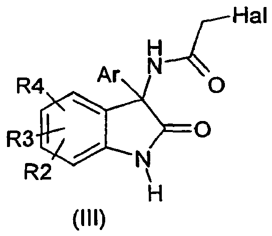 avec un composé de formule générale (IV) : 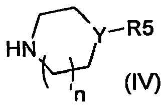 dans lesquelles Y, R2, R3, R4, R5, Ar, n sont tels que définis en formule générale (I). Cette réaction est généralement effectuée au moyen d'une base, organique ou minérale, telle que K₂CO₃, Na₂CO₃, la pyridine ou 4-diméthylaminopyridine, en présence de Nal ou Kl, dans un solvant inerte tel que le DMF, le dichlorométhane, le THF, le diméthoxyéthane ou le toluène.

Le composé de formule générale (III) peut être préparé à partir d'un composé de formule générale (V) : 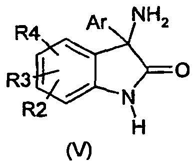 et d'un composé de formule générale (VI) : 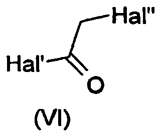 dans lesquelles R2, R3, R4, R5, Ar sont tels que définis en formule générale (I) et Hal' et Hal", identiques ou différents représentent indépendamment un atome d'halogène, de préférence le chlore.

Cette réaction est généralement effectuée au moyen de pyridine ou 4-diméthylaminopyridine dans un solvant tel que le toluène, benzène ou dichlorométhane, préférentiellement à température comprise entre la température ambiante et la température de reflux du solvant.

Lorsque X=-CH, -N< ou 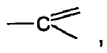 le composé de formule générale (I) dans laquelle R1=H peut également être préparé à partir d'un composé de formule générale (V) : 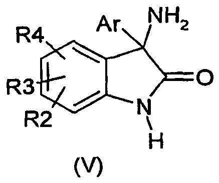 et d'un composé de formule générale (VII) : 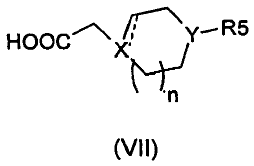 dans lesquelles 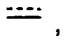 R2, R3, R4, R5, Ar, X, Y et n sont tels que définis en formule générale (I) Cette réaction est généralement effectuée au moyen d'un agent d'halogénation, tel qu'un agent de chloration, par exemple les chlorures de phosphore, notamment PCl₅, ou encore PCl₃ ou POCl₃. La réaction est généralement réalisée en présence de pyridine ou 4-diméthylaminopyridine, dans un solvant tel que le dichlorométhane ou le DMF.

Les intermédiaires de formule générale (V) sont connus et peuvent être préparés selon les procédés illustrés par le schéma qui suit : 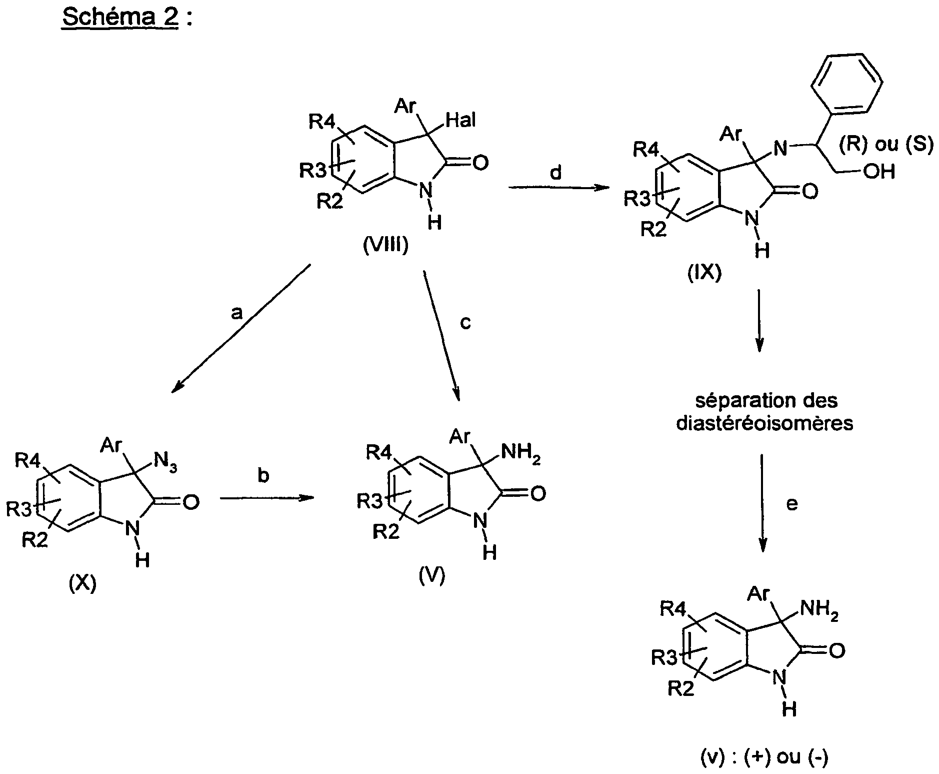 dans lequel R2, R3, R4 et Ar sont tels que définis en formule générale (I) et Hal représente un atome d'halogène, par exemple le chlore.

A l'étape c du Schéma 2, on prépare un composé de formule (V) à partir d'un composé de formule (VIII) par barbotage d'ammoniac gazeux selon la méthode décrite dans la demande FR 2 714 378.

On peut également préparer le même composé par réduction d'un composé de formule (X) selon des méthodes connues de l'homme du métier, par exemple au moyen du zinc dans un solvant tel que le méthanol. La préparation d'un composé de formule (X) de l'étape est décrite dans la demande FR 2 714 378.

Un composé de formule (V) optiquement pur peut être synthétisé selon les étapes d et e du Schéma 3, telles que décrites dans la demande WO 03/008407.

Les intermédiaires de formule générale (VIII) peuvent être préparés selon les procédés décrits dans la demande WO 03/008407 et illustrés par le schéma 3 : 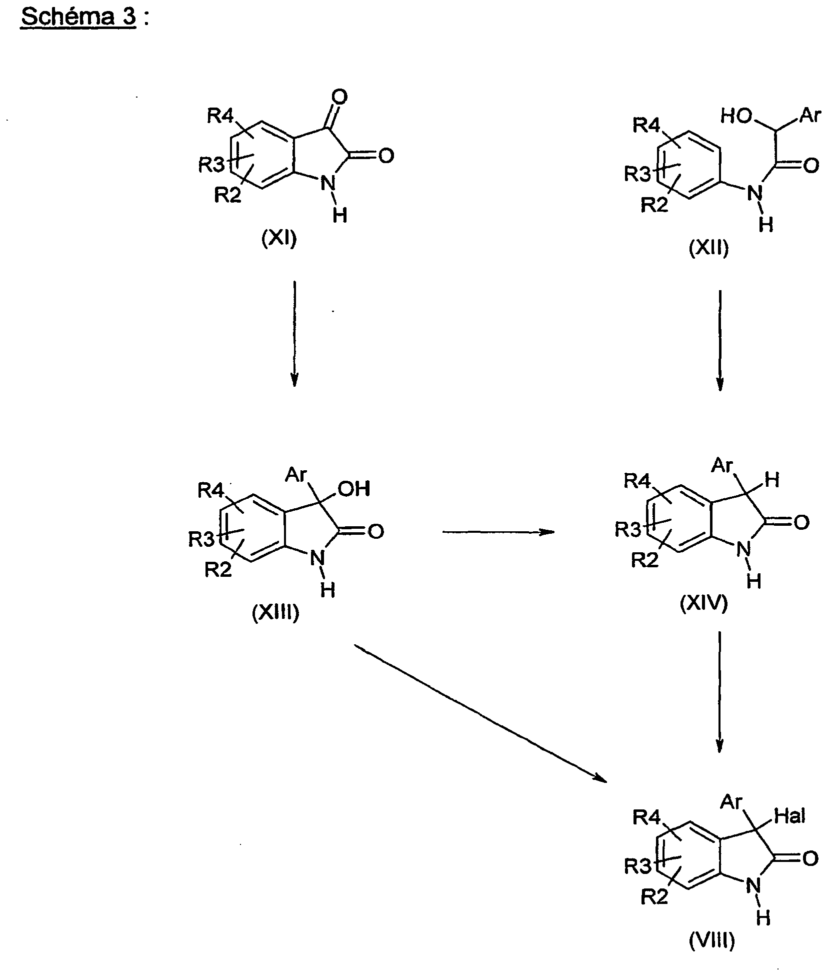 dans lequel R2, R3, R4 et Ar sont tels que définis en formule générale (I) et Hal représente un atome d'halogène, par exemple le chlore.

Les composés de formule générale (XIV) peuvent également être préparés par application ou adaptation des procédures décrites dans Journal of Heterocyclic Chemistry, 43(4), 1031-1035; 2006.

Ainsi les composés de formule générale (XIV) peuvent être préparés par réduction d'un composé de formule (XV): 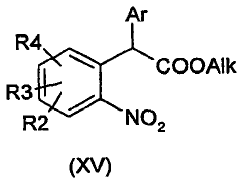 dans laquelle R2, R3, R4 et Ar sont tels que définis en formule générale (I) et Alk représente un groupe alkyle par exemple un groupe méthyle, éthyle ou butyle. Généralement cette réaction est réalisée au moyen de fer, en milieu acide, par exemple au moyen d'acide acétique.

Le composé de formule générale (XV) peut être obtenu par réaction d'un composé de formule (XVI): 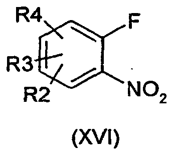 dans laquelle R2, R3 et R4 sont tels que définis en formule générale (I) avec un composé de formule (XVII) : 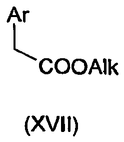 dans laquelle Ar est tel que défini en formule générale (I) et Alk représente un groupe alkyle, par exemple un groupe méthyle, éthyle ou butyle, selon des méthodes connues de l'homme du métier et décrites dans Journal of Heterocyclic Chemistry, 43(4), 1031-1035; 2006, par exemple en présence d'une base telle que t-BuOK or NaH.

Le composé de formule générale (VII) peut être préparé selon l'une ou l'autre des méthodes suivantes, illustrées par le schéma 4 :

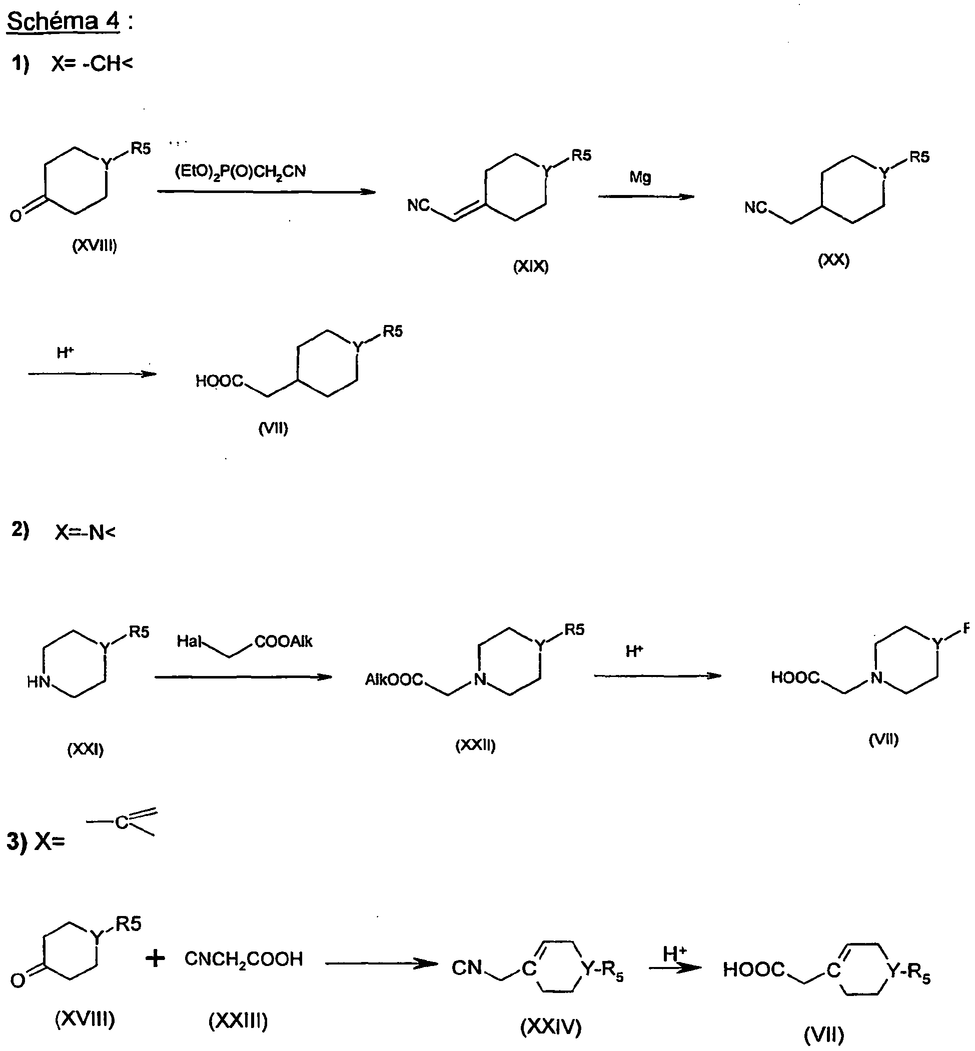

Selon le premier mode de réalisation, lorsque X=-CH<, le composé de formule (VII) peut être préparé par hydrolyse d'un composé de formule (XX) : 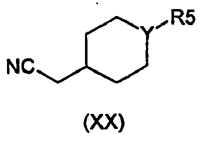 dans laquelle R5 et Y sont définis comme en formule générale (I), en milieu acide; par exemple au moyen d'acide chlorhydrique concentré.

Le composé de formule générale (XX) peut être préparé par réduction d'un composé de formule (XIX) : 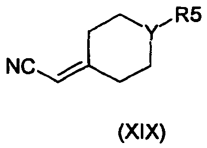 dans laquelle R5 et Y sont définis comme en formule générale (I), par exemple au moyen de magnésium. Cette réaction est généralement effectuée dans un solvant tel que le méthanol ou l'éthanol.

Le composé de formule générale (XIX) peut être préparé par réaction de Wittig-Hömer à partir du composé de formule générale (XVIII) 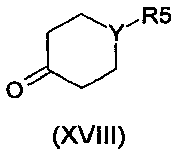 dans laquelle R5 et Y sont définis comme en formule générale (I). Généralement, cette réaction est réalisée au moyen d'un dérivé phosphonate approprié, tel que le diéthyle (cyanométhyle)phosphonate. On opère avantageusement en présence d'une base, telle que K₂CO₃ dans un solvant tel que le THF ou diméthoxyéthane.

Selon le second mode de réalisation, lorsque X=-N<, le composé de formule (VII) peut être préparé à partir d'un composé de formule (XXII) : 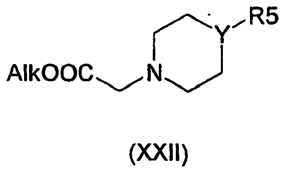 dans laquelle R5 et Y sont définis comme en formule générale (I), et , représente un groupe alkyle par exemple un groupe méthyle, éthyle ou butyle. Cette réaction est généralement effectuée en milieu acide, par exemple au moyen d'acide chlorhydrique concentré.

Le composé de formule générale (XXII) peut être préparé par condensation d'un composé de formule générale (XXI) : 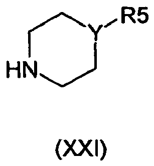 dans laquelle R5 et Y sont définis comme en formule générale (I), avec un composé halogéné correspondant, tel que Hal"'CH₂COOAlk, où Hal"' représente un atome d'halogène, tel que le chlore et Alk représente un groupe alkyle, tel que éthyle. Cette réaction est avantageusement effectuée dans un solvant tel que le toluène ou le benzène ou le dioxane.

Selon le troisième mode de réalisation, lorsque X= 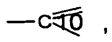 le composé de formule (VII) peut être préparé par hydrolyse d'un composé de formule (XXIV) : 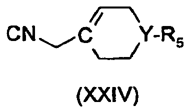 dans laquelle R5 et Y sont définis comme en formule générale (I), en milieu acide, par exemple au moyen d'acide chlorhydrique concentré.

Le composé de formule générale (XXIV) peut être préparé par réaction à partir du composé de formule générale (XVIII) 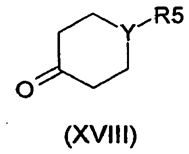 dans laquelle R5 et Y sont définis comme en formule générale (I), au moyen du composé de formule générale (XXIII) :

NC-CH₂COOH (XXIII)

Généralement, cette réaction est réalisée dans un solvant tel que le THF.

Selon un autre mode de réalisation les composés de formule générale (I) dans laquelle R1 représente un group alkyle et R2, R3, R4, R5, Ar, X, Y et n sont tels que définis en formule générale (I) peuvent également être préparés selon le schéma 5 suivant :

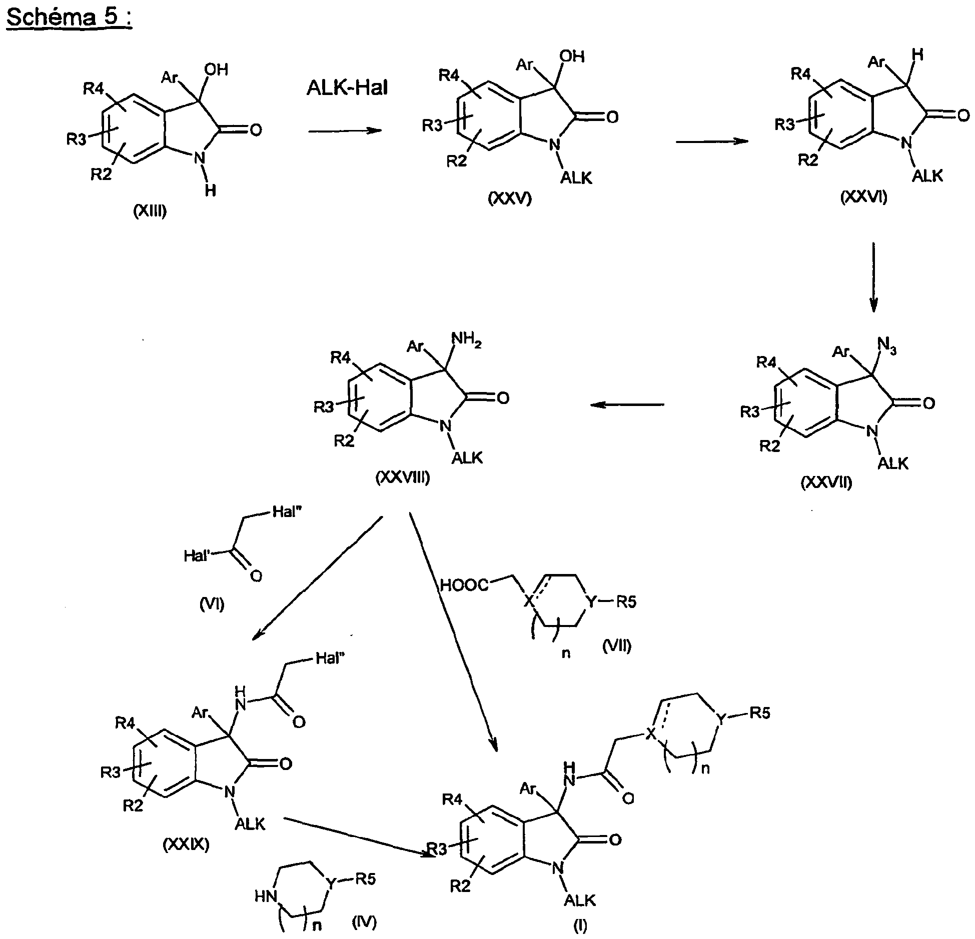

Le composé de formule générale (XXV) peut être préparé par réaction d'un composé de formule (XIII) avec un composé de formule (XIV)

ALK-Hal (XIV),

dans laquelle ALK représente un groupe aliphatique saturé linéaire ou ramifié comprenant de 1 à 6 atomes de carbone et Hal représente un atome d'halogène, par exemple le chlore selon des méthodes connues de l'homme du métier par exemple en présence d'une base, telle que K₂CO₃, NaH, t-BuO⁻K⁺ dans un solvant tel que le DMF, THF, diméthoxyéthane, DMSO.

Les composés de formule générale (XXVIII) peuvent être préparés selon des méthodes analogues à celles décrites précédemment.

Les composés de formule générale (XXVIII) peuvent également être préparés selon le schéma 6 suivant :

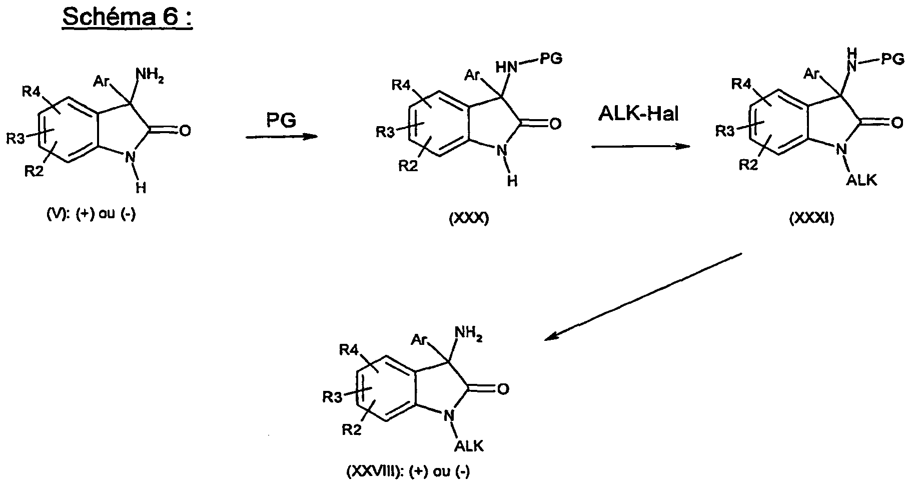

Selon ce schéma, on fait réagir un composé de formule (V) avec un groupe protecteur PG pour donner le composé de formule (XXX). En tant que groupe protecteur PG de l'amine, on peut utilsier par exemple la benzimine ou le t-butyl carbamate. Ces derniers sont introduits selon des méthodes connues de l'homme du métier par exemple en présence d'une base, telle que K₂CO₃, NaOH, triéthylamine dans un solvant tel que le dioxane, THF ou le DMSO.

Le composé de formule générale (XXXI) peut être préparé par réaction d'un composé de formule (XXX) avec un composé de formule (XIV)

ALK-Hal (XIV),

dans laquelle ALK représente un groupe aliphatique saturé linéaire ou ramifié comprenant de 1 à 6 atomes de carbone et Hal représente un atome d'halogène, par exemple le chlore.

On obtient le composé de formule générale (XXVIII) à partir d'un composé de formule (XXXI) par élimination du groupe protective selon des méthodes bien connues, par exemple en milieu acide par HCl ou acide trifluoroacétique.

En opérant ensuite par application des méthodes décrites ci-dessus pour les composés de formule générale (XIII), (XIV), (X), (V), (III) et (I) des schéma 1, 2 et 3 on obtient les composés de formule générale (XXV), (XXVI), (XXVII), (XXVIII), (XXIX) et (I).

Eventuellement, on transforme le composé de formule (I) en l'un de ses sels d'addition à un acide.
Le procédé selon l'invention peut éventuellement comprendre l'étape consistant à isoler le produit de formule générale (I) désiré.

Dans les schémas 1, 2, 3, 4, 5 et 6, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

L'invention, selon un autre de ses aspects, a également pour objet les composés de formule (III), à l'exclusion du 5-chloro-3-(2-chloroacétamido)-3-(2-chlorophényl)-1,3-dihydroindol-2-one. Ces composés sont utiles comme intermédiaires de synthèse des composés de formule (I).

Parmi les composés de formule (III) objets de l'invention, un groupe de composés est constitué par les composés pour lesquels :
Ar représente un groupe hétéroaryle éventuellement substitué par un ou plusieurs substituants, identiques ou différents, choisis parmi les atomes d'halogène, les groupes (C1-6) alkyle, perhalogéno(C1-3) alkyle, (C1-6) alcoxy, aryle.

L'invention, selon un autre de ses aspects, a également pour objet les composés de formule (XXVIII) et (XXIX). Ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Les mesures physico-chimiques ont été effectuées de la façon suivante : Les points de fusion ont été mesurés avec un appareil BUCHI B-540.

Les spectres de résonance magnétique nucléaire du proton (RMN 1H) ont été enregistrés à 500 MHz sur un appareil Bruker équipé avec une console Avance III. Les déplacements chimiques sont rapportés en ppm par rapport à la fréquence du TMS.
Touts les spectres ont été enregistrés à la température de 40°C
Les abréviations utilisées pour caractériser les signaux sont les suivantes:
s = singulet, sb =singulet large, m = multiplet, d = doublet, t = triplet, q = quadruplet.
* = non intégrable à cause de l'interférence avec un pic large dû à l'eau.
** = non intégrable à cause de l'interférence avec un pic dû au solvant RMN.

L'HPLC a été effectuée au moyen d'un système ThermoElectron Surveyor équipé avec un détecteur à spectrométrie de masse à trappe d'ions ainsi que d'un détecteur à barre de diodes.
Les conditions d'analyse par chromatographie liquide couplée à la spectrométrie de masse (LC/UV/MS) sont les suivantes:
Pour la partie chromatographie liquide trois systèmes chromatographiques différents sont utilisés:
- système chromatographique A
   - Eluant A = H₂O + 0,005% TFA
   - Eluant B = CH₃CN.
   - Gradient de 95% de A à 90% de B en 17 minutes, puis élution par 90% de B pendant 5 minutes.
   - Débit 0,3ml/minute
   - Injection de 2µL de solution à 0,1mg/ml dans un mélange CH₃CN : H₂O = 9:1
- système chromatographique B
   - Eluant A = H₂O + 0,01% TFA
   - Eluant B = CH₃CN
   - Gradient de 98% de A à 95% de B en 10 minutes, puis élution par 95% de B pendant 5 minutes.
   - Débit 0,5ml/minute; température 40°C
   - Injection de 2µL de solution à 0,1mg/ml dans un mélange CH₃CN : H2O = 9:1
- système chromatographique C
   - Eluant A = H₂O + 0.005 M Ammonium Acetate pH 6.5
   - Eluant B = CH₃CN.
      Gradient de 95% de A à 90% de B en 17 minutes, puis élution par 90% de B pendant 5 minutes.
   - Débit 0,3ml/minute
   - Injection de 2µL de solution à 0,1mg/ml dans un mélange CH₃CN : H2O = 9 :1

Les colonnes utilisées sont :
- colonne Waters XTerra MS C18 2,1 x 50 mm 3,5 µm n°186000400
- colonne Waters XBridge C18 2.1x50 mm 2.5 µm n°186003085
- colonne Phenomenex Gemini C18 2.1x100 mm 5.0 µm n°00D-4435-B0
- colonne Waters Sunfire C18 2,1x100 mm 3,5 µm n°186002534

Les produits sont détectés en UV à 220 nm.

Pour la partie spectrométrie de masse :
- Mode d'ionisation: électrospray positif (API-ES polarité+)
- Balayage de 100 à 1200 uma

La chromatographie en couche mince a été effectuée sur des plaques CCM de gel de silice de Merck. Le gel de silice pour la chromatographie sur colonne flash est commercialisé par Biotage.
Tous les solvants utilisés sont de pureté "reagent grade" ou "HPLC grade".

Les mesures de Alpha D ont été effectuées sur un polarimétre Perkin Elmer modéle PE341 en utlilisant une cellule avec un chemin optique de 1 dm.

Dans les exemples et préparations :
AcOH et AcOEt représentent respectivement l'acide acétique et l'acétate d'éthyle.
MeOH, EtOH, tBuOH représentent respectivement méthanol, éthanol et ter-butanol.
Pf représente point de fusion.

### Préparation 1:

### Acide (1-méthyl-piperidin-4-yl) acétique

### (i) (1-Méthyl-piperidin-4-ylidene)-acétonitrile

Dans un ballon, muni d'un agitateur magnétique et sous flux d'azote, on charge 9,36 g de K₂CO₃, 8,89 ml de diéthyle(cyanométhyle)phosphonate dans 12 ml de THF et on laisse réagir 15 minutes à température ambiante puis on porte à reflux pendant 20 minutes. On laisse refroidir et on ajoute goutte à goutte 6,5 ml de 1-méthyle-4-pipéridone. On laisse à reflux pendant 16 heures. On verse ensuite le mélange réactionnel dans l'eau et on extrait à l'acétate d'éthyle. La phase organique est séchée sur Na₂SO₄, filtrée et évaporée sous vide. On obtient 6,8 g d'huile.

### (ii) (1-Méthyl-piperidin-4-yl)-acétonitrile.

Dans un ballon, muni d'un agitateur magnétique, on charge 1 g du produit obtenu à l'étape précédente dans 70 ml de méthanol. A 0°C, on ajoute à petites portions 7,2 g de magnésium. On laisse sous agitation pendant 4 heures. On filtre pour éliminer les particules solides de magnésium et on évapore les eaux de filtration. On reprend le résidu avec une solution saturée de NaCl et on extrait avec le dichlorométhane. La phase organique est séchée sur Na₂SO₄, filtrée et évaporée sous vide. On obtient 450 mg d'huile.
CCM: AcOEt 9 / MeOH 1, Rf=0.2

### (iii) Acide (1-méthyl-piperidin-4-yl) acétique

Dans un ballon, muni d'un agitateur magnétique, on charge 3,65 g du produit obtenu à l'étape (ii) dans 47 ml d'acide chlorhydrique concentré. On laisse à reflux pendant 20 heures. On dilue avec de l'eau et on extrait avec le dichlorométhane pour éliminer les impuretés. On porte la phase aqueuse à pH 5-6 et on extrait au dichlorométhane. On évapore la phase aqueuse sous vide et on isole un solide blanc. On reprend avec de l'éthanol pour séparer le produit des sels. On évapore les eaux de filtration pour isoler 3,6 g de solide jaune pâle.
CCM : MeOH 99/ NH₄OH 1, Rf=0.2

### Préparation 2:

### Acide (1-éthyl-piperidin-4-yl) acétique

En opérant comme décrit dans la Préparation 1 mais en utilisant le 1-éthyle-4-pipéridone au lieu 1-méthyle-4-pipéridone on obtient le composé du titre.
CCM : 100% MeOH, Rf=0.15 ;

### Préparation 3:

### Acide (4-éthyl-pipérazin-1-yl) acétique

### (i) éthyle (4-éthyl-pipérazin-1-yl)-acétate

Dans un ballon, on charge 8,9 ml d'éthylpipérazine dans 91,5 ml de toluène. On ajoute goutte à goutte une solution de 4,1 ml de bromoacétate d'éthyle dans 11,6 ml de toluène. On laisse réagir à reflux à 110°C pendant une heure, on concentre à petit volume et on laisse au frigo pendant 3 heures. Il se forme un précipité blanc qui est filtré et lavé avec du dichlorométhane. On évapore les eaux de filtration; on obtient 7 g de produit attendu.
CCM : AcOEt 1/ MeOH 1, Rf=0.45

### (ii) Acide (4-éthyl-pipérazin-1-yl) acétique

On ajoute 7 g du produit obtenu à l'étape précédente à 190 ml de HCl 6N et on laisse réagir 4 heures à reflux. On évapore à sec, on lave avec un mélange AcOEt 1 / EtOH 1 et on sèche le solide blanc obtenu. On obtient 7 g de produit attendu.
CCM: 100% MeOH, Rf=0.2

### Préparation 4:

### Acide (4-méthyl-pipérazin-1-yl) acétique

En opérant comme décrit dans la Préparation 3 mais en utilisant la 1-méthylepipérazine au lieu de la 1-éthylepipérazine on obtient le composé du titre.
CCM: 100% MeOH, Rf= 0.25 ;

### Préparation 5:

### (+) 3-Amino-5,6-dichloro-1,3-dihydro-3-(4-chlorophényl)-indole-2-one

### (i) 5,6-dichloro-1,3-dihydro-3-(4-chlorophényl)-indole-2-one

### A) Méthyl-(4-chlorophényl)-(4,5-dichloro-2-nitrophényl)-acétate

On ajoute sous flux d'azote à une suspension à -10°C de 2,85 g de NaH à 60% dans 45 ml de DMF une solution de 5 g de 1,2-dichloro-4-fluoro-5-nitrobenzène et 4,4g de méthyl-4-chloro-phénylacétate dans 70 ml de DMF et on maintient la température à -5°C. On laisse réagir pendant 2 heures en laissant la température remonter à température ambiante. On verse sur de la glace, on ajoute une solution aqueuse de NH₄Cl à 10% et on extrait à l'acétate d'éthyle. La phase organique est séchée, filtrée et concentrée. On obtient 30 g d'huile brune que l'on purifie sur colonne avec Hex. puis un mélange Hex. /AcOEt 95/5 pour obtenir 3,18 g d'huile.

### B) 5,6-dichloro-1,3-dihydro-3-(4-chlorophényl)-indole-2-one

Dans un ballon muni d'un agitateur mécanique, sous flux d'azote, on charge 4,6 g du produit de l'étape A, 60 ml de méthanol, 15 ml de AcOH, 2,7 g de fer et on laisse à reflux pendant 1h30. On verse sur de la glace et on ajoute une solution de NaHCO₃ à 10% jusqu'au pH basique. On ajoute de l'acétate d'éthyle et on filtre. On sépare la phase organique que l'on sèche, filtre et concentre. On obtient un solide que l'on reprend à l'isopropyléther et on filtre. On obtient 2,75 g de solide blanc.
Pf : 214-215°C

### (ii) 5,6-dichloro-[[(1R)-2-hydroxy-1-phényléthyl]amino]1,3-dihydro-3-(4-chlorophényl)-indole-2-one isomère A et isomère B

### A) 3-bromo-5,6-dichloro-1,3-dihydro-3-(4-chlorophényl)-indole-2-one

Sous flux d'azote on dissout 2,75g du produit de l'étape B précédente dans 100 ml de dichlorométhane. On refroidit dans un bain de glace et on ajoute goutte à goutte une solution de 3,93 g de PhMe₃NBr₃ dans 100 ml de dichlorométhane. On laisse réagir pendant 3 heures et on laisse remonter la température progressivement à température ambiante. On lave avec de l'acide chlorhydrique 1 M et de l'eau. On sèche, filtre et concentre. On obtient 3,7 g d'huile.

### B) 5,6-dichloro-[[(1R)-2-hydroxy-1-phényléthyl]amino]1,3-dihydro-3-(4-chlorophényl)-indole-2-one isomère A et isomère B

Sous flux d'azote, on mélange 3,4 g du composé de l'étape précédente dans 50 ml de chloroforme et 2,9 g de R-phénylglycynol. On laisse réagir 2 heures à température ambiante puis on ajoute 1,6 ml de DIPEA et on laisse agir à température. On filtre le solide formé, les eaux de filtration sont évaporées à sec et purifiées sur colonne avec l'éluant Hex. /AcOEt 7:3].

On obtient 1,8 g de produit moins polaire, isomère A (pf=79.8-80.5°C) et 2,2 g de l'isomère plus polaire B (pf=213.2°C)

### (iii) (+)-3-Amino-5,6-dichloro-1,3-dihydro-3-(4-chlorophényl)-indole-2-one

On met à réagir 1,8 g du produit obtenu dans l'étape précédente dans un mélange de 28 ml de dichlorométhane et 12 ml de méthanol. On ajoute 1,9 g de Pb(OAc)₄ et on laisse réagir à température ambiante pendant 3 heures. On évapore à sec et on reprend avec de l'acétate d'éthyle puis on lave avec une solution aqueuse saturée de NaHCO₃. La phase organique est séchée, filtrée et concentrée. On reprend avec un mélange de 36 ml d'acide chlorhydrique 3N et 3,7 ml de méthanol et on laisse sous agitation pendant une nuit. On concentre et on dilue avec un mélange d'eau et de dichlorométhane. La phase organique est lavée avec une solution d'acide chlorhydrique 1 N. Les phases aqueuses sont réunies, portées à pH basique avec une solution de NH₃ aqueuse, et extraites avec du dichlorométhane. La phase organique est séchée, filtrée et concentrée pour obtenir 540 mg de produit solide blanc.
Pf=221°C ; alphaD=+32,5°, c=0.5 wt% MeOH

### Préparation 6:

### (-) 3-Amino-5,6-dichloro-1,3-dihydro-3-(4-chlorophényl)-indole-2-one

En opérant comme décrit dans la Préparation 5 (iii) mais en utilisant l'isomère B plus polaire obtenu à la Préparation 5(ii) au lieu de l'isomère A de la même préparation, on obtient le composé du titre.
Alpha D= -23.6°, c=0.35 wt% MeOH.

### Préparation 7:

### 3-Amino-4,6-dichloro-1,3-dihydro-3-(4-chlorophényl)-indole-2-one

### (i) 4,6-dichloro-1,3-dihydro-3-(4-chlorophényl)-indole-2-one

### a) 4-chloro-O-acyl-mandelic-chlorure

Dans un ballon à deux cols muni d'un agitateur magnétique, on charge 10 g d'acide 4-chloro-dl-mandélique, 88 ml de dichlorométhane, 4,2 ml de chlorure d'acétyle. On fait réagir à 50° pendant 3 heures. Puis on ajoute 7,8 ml de chlorure de thionyle. On laisse réagir à reflux pendant 2 heures. On évapore sous vide et on obtient 13,7 g d'un liquide opaque.

### b) 4-chloro-N-3,5-phényl-mandelammide

Dans un ballon à trois cols muni d'un agitateur mécanique et sous flux d'azote, on charge 4,04 g de 3,5-dichloroaniline, 50 ml de toluène. On porte à 0°C. On ajoute alors 9,6 g de carbonate de potassium. On ajoute lentement 6,8 g du produit obtenu à l'étape précédente dilué dans 10 ml de toluène. On laisse réagir à température ambiante pendant une heure puis on ajoute 4,15 mL de méthanol. On laisse réagir à 80° pendant 2 heures puis on verse une solution d'acide chlorhydrique 1 N et on extrait à l'acétate d'éthyle. La phase organique est évaporée sous vide. On obtient 5,7 g de solide.

### c) 4,6-dichloro-1,3-dihydro-3-(4-chlorophényl)-indole-2-one

Dans un ballon muni d'un agitateur magnétique, on charge 22 ml d'acide sulfurique à 96% et 5 ml d'acide sulfurique fumant. On rafraîchit dans un bain de glace et on ajoute par petites portions 5,7 g du produit obtenu à l'étape précédente. On laisse ensuite réagir à température ambiante pendant 4 heures. On verse la réaction dans un bain de glace et on porte à pH basique avec une solution de NaHCO₃ puis avec une solution de soude concentrée. On extrait avec du dichlorométhane, on sépare la phase organique que l'on sèche sur Na₂SO₄, filtre et évapore sous vide pour obtenir 7,5 g d'un solide que l'on reprend dans l'éther éthylique. On filtre et on obtient 4,2 g de poudre.

### (ii) 3-Azido-4,6-dichloro-1,3-dihydro-3-(4-chlorophényl)-indole-2-one

### A) 3-bromo-4,6-dichloro-1,3-dihydro-3-(4-chlorophényl)-indole-2-one

On obtient ce produit en opérant comme décrit au point (ii) à de la Préparation 5 mais en utilisant le composé obtenu à l'étape précédente.

### B) 3-Azido-4,6-dichloro-1,3-dihydro-3-(4-chlorophényl)-indole-2-one

Dans un ballon à trois cols muni d'un agitateur mécanique et sous flux d'azote, on charge 550 mg du composé obtenu à l'étape précédente, dans 17 ml d'acétonitrile et 270 mg de NaN₃. On porte à reflux pendant 2 heures et on reprend à l'acétate d'éthyle, on lave avec une solution saturée de chlorure de sodium. On sépare la phase organique, qui est séchée sur Na₂SO₄, filtrée et évaporée sous vide pour obtenir 320 mg d'une résine qui est purifiée par flash chromatographie avec l'éluant cyclohexane/acétate d'éthyle 85/15. On évapore la phase contenant le produit pour obtenir 220 mg d'un solide blanc.

### (iii) 3-Amino-4,6-dichloro-1,3-dihydro-3-(4-chlorophényl)-indole-2-one

Dans un ballon à deux cols muni d'un agitateur mécanique, on charge 220 mg du produit obtenu à l'étape précédente dans 5 ml de THF, 10 ml de méthanol, 170 mg de NH₄Cl, 80 mg de zinc. On laisse réagir à température ambiante pendant 3 heures. On filtre, le résidu est évaporé sous vide. On reprend avec de l'acétate d'éthyle, et on lave avec une solution saturée de chlorure de sodium. On sépare la phase organique qui est séchée sur Na₂SO₄, filtrée et évaporée sous vide. On obtient 200 mg d'huile qui est purifiée par flash chromatographie avec l'éluant cyclohexane/acétate d'éthyle 8/2. On obtient 74 mg de produit attendu].
CCM: Cyclohexane 6/ AcOEt 4, Rf=0.3

### Préparation 8:

### (+)3-Amino-4,6-dichloro-1,3-dihydro-3-(4-trifluorométhylphényl)-indole-2-one

### (i) 3-Hydroxy-4,6-dichloro-1,3-dihydro-3-(4-trifluorométhylphényl)-indole-2-one

Dans un ballon muni d'un agitateur mécanique et sous flux d'azote, on charge 1,8 g de magnésium pour Grignard dans 19 ml d'éther éthylique anhydre. Puis on ajoute un mélange de 8,9 ml de 4-bromotrifluorométhylbenzène dans 46 ml d'éther éthylique anhydre. On laisse sous agitation pendant une heure, puis on ajoute une solution de 5,7 g de 4,6-dichloro-1H-indol-2,3-dione dans 100 mL de THF anhydre. On laisse agiter à température ambiante pendant 4 heures et demie. On ajoute de l'eau, et on extrait avec de l'acétate d'éthyle. On sépare la phase organique que l'on sèche sur Na₂SO₄, filtre et évapore sous vide. On reprend avec de l'acétate d'éthyle et on lave avec une solution de soude 1 N. La phase organique est séchée sur Na₂SO₄, filtrée et évaporée sous vide. Le solide est repris avec de l'éther éthylique et filtré. On obtient 4,7 g de produit attendu.

### (ii) 3,5,6-trichloro-1,3-dihydro-3-(4-trifluorométhylphényl)-indole-2-one

Dans un ballon muni d'un agitateur magnétique et sous flux d'azote, on charge 1,2 g du produit de l'étape précédente, dans 8 ml de dichlorométhane. A 0°C, on ajoute 0,47 ml de pyridine et un mélange de 0,34 ml de SOCl₂ dans 4 ml de dichlorométhane. On laisse réagir à température ambiante, puis on verse dans une solution aqueuse saturée de NH₄Cl. On sépare la phase organique qui est séchée sur Na₂SO₄, filtrée et évaporée sous vide.
CCM: Hex.1/ AcOEt 1, Rf=0.85

### (iii) 4,6-dichloro-[ [(1S)-2-hydroxy-1-phényléthyl] amino] 1,3-dihydro -3-(4-trifluoromethyl -phényl)-indole-2-one isomère A et isomère B

En opérant comme décrit à l'étape (ii) A et B de la Préparation 5 mais en utilisant le composé de l'étape précédente et le (S) phénylglycinole au lieu du (R) phénylglycinole on obtient les composées du titre.
CCM: AcOEt 4/ cyclohexane 6, Rf=0.5 (isomère A), Rf=0.2 (isomère B)

### (iv) (+) 3-Amino-5,6-dichloro-1,3-dihydro-3-(4-trifluorométhylphényl)-indole-2-one

On obtient ce composé en opérant comme décrit à l'étape (ii) B de la Préparation 5.
Alpha D= +60°, c=0.25 wt% in MeOH

### Préparation 9:

### 3-Amino-1,5-diméthyl-6-chloro-1,3-dihydro-3-(4-chlorophényl)-indole-2-one

### (i) 6-Chloro-3-(4-chloro-phényl)-3-hydroxy-1,5-diméthyl-1,3-dihydro-indol-2-one

On prépare ce composé à partir du 3-Hydroxy-5-méthyl-6-chloro-1,3-dihydro-3-(4-chlorophényl)-indole-2-one (composé obtenu selon le procédé décrit au point (i) de la Préparation 8), on en dissout 1,2 g dans 8 ml de DMF. On ajoute à 0°C, sous flux d'azote167 mg de NaH à 60%. Puis on ajoute 260 µl de CH₃I et on laisse réagir pendant 30 minutes. On verse dans l'eau et on extrait au dichlorométhane. La phase organique est séchée, filtrée et concentrée. On purifie sur colonne avec l'éluant Hex. /AcOEt 9/1
CCM: AcOEt 1/ Hex. 1, Rf=0.7

### (ii) 6-Chloro-3-(4-chloro-phényl)-1,5-diméthyl-1,3-dihydro-indol-2-one

On mélange 1 g du produit obtenu à l'étape précédente dans 4 ml de TFA et 1,3 ml de HSiEt₃ et on laisse réagir pendant une heure à 80°C. On verse dans l'eau et on porte à pH basique avec une solution aqueuse de NH₃. On extrait avec de l'acétate d'éthyle. La phase organique est séchée, filtrée et concentrée. On reprend avec de l'éther éthylique et on filtre. On obtient 688 mg d'un solide blanc.
CCM: AcOEt 4/ Hex. 6, Rf=0.7

### (iii) 3-Amino-1,5-diméthyl-6-chloro-1,3-dihydro-3-(4-chlorophényl)-indole-2-one

En opérant comme décrit dans la Préparation 7 aux points (ii) et (iii) mais en utilisant le produit de l'étape précédent au lieu du produit obtenu à l'étape (i) de la Préparation 7 on obtient le composé du titre.
CCM: AcOEt 1/ Hex. 1, Rf=0.5

### Préparation 10:

### Acide (1-éthyl-1,2,3,6-tetrahydropyrid-4-yl) acétique

### (i) (1-éthyl-1,2,3,6-tetrahydropyrid-4-yl)-acétonitrile.

Dans un ballon, muni d'un agitateur magnétique et sous flux d'azote, on charge 2,9 g de 1-éthyl-4-pipéridone, 3,3 g d'acide cyanacétique et 36 ml de toluène. On porte à reflux pendant 4 heures on éliminant l'eau à l'aide d'un appareil de Markusson. On évapore sous vide. On obtient 4,2 g d'huile.

### (ii) Acide (1-éthyl-1,2,3,6-tetrahydro-pyrid-4-yl) acétique

En opérant comme décrit dans la Préparation 1 (iii) mais en utilisant le produit de l'étape précédente au lieu du produit de la préparation 1 (ii), on obtient le composé du titre.
CCM : MeOH 99/ NH₄OH 1, Rf=0.2
RMN ¹H : α (ppm, dmso-d6): 1.09 (t, J= 7.2Hz, 3H), 2.18 (m, 2H), 2.62 (q, J= 7.2Hz, 2H), 2.68 - 2.76 (m, 2H), 2.92 (s, 2H), 3.11 (sb, 2H), 5.49 (m, 1 H).

### Préparation 53:

### (+) 3-amino-1-isopropyl-4,6-dichloro-1,3-dihydro-3-(3,4-dichlorophényl)-indole-2-one

### (i) (+)3-amino-4,6-dichloro-1,3-dihydro-3-(3,4-dichlorophényl)-indole-2-one

On obtient le composé en opérant comme décrit à la Préparation 5 mais en utilisant à l'étape (i) le 3,4-dichloro-bromobenzene au lieu du 4-bromotrifluorométhylbenzène.

### (ii) (+)3-benzimino-4,6-dichloro-1,3-dihydro-3-(3,4-dichlorophényl)-indole-2-one

On charge dans un ballon 215 mg du produit obtenu à l'étape précédente ainsi que 120µl de benzaldéhyde. On chauffe à 100°C pendant 5 min dans un réacteur à micro-ondes. On obtient un solide qui est séché sous vide pour éliminer le benzaldéhyde.

### (iii) (+)3-benzimino-1-isopropyl-4,6-dichloro-1,3-dihydro-3-(3,4-dichlorophényl)-indole-2-one

On charge dans un ballon 144mg du produit obtenu à l'étape précédente dans du DMF (800 µl) et en présence de K2CO3 (49mg) et du bromure d'isopropyle (30 µl).On chauffe à 140°C pendant 5 min dans un réacteur à micro-ondes. On filtre et on utilise le produit brut pour l'étape suivante.

### (iv) (+)3-amino-1-isopropyl-4,6-dichloro-1,3-dihydro-3-(3,4-dichlorophényl)-indole-2-one

On dissout le produit obtenu à l'étape précédente (157mg) dans du méthanol (550 µl) et on ajoute 2,7ml d'une solution 3N d'acide HCl. Après 5 heures à température ambiante, on neutralise avec de l'ammoniaque et on extrait avec de l'acétate d'éthyle. On sèche et on évapore. On obtient un produit semi-solide (112mg).

Les intermédiaires suivants de formule générale (V) dans laquelle R1, R2, R3, R4 et Ar sont tels que définis dans le Tableau 1, ont également été préparés avec les méthodes utilisés pour les Préparations 5, 6, 7, 8 et 9.
Le tableau 1 qui suit illustre les structures chimiques et les propriétés physiques de quelques préparations selon l'invention. Dans ce tableau :
- dans la colonne « isomère », « rac » représente un mélange racémique, et (+) ou (-) représente l'un ou l'autre des stéréoisomères,
- Me, Et, n-Pr, i-Pr, n-Bu et i-Bu représentent respectivement des groupes méthyle, éthyle, n-propyle, isopropyle, n-butyle et isobutyle, et
- Ph et Bn représentent respectivement des groupes phényle et benzyle.

**TABLEAU 1**

| | | | | | | |
|---|---|---|---|---|---|---|
| 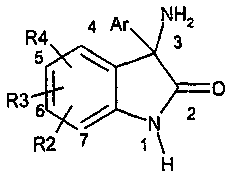 | | | | | | |

| Préparation | Ar | R2 | R3 | R4 | isomère | Analyses |
|---|---|---|---|---|---|---|
| 11 | 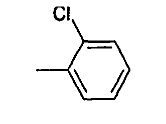 | 6-Cl | 5-ME | H | rac | (M+H)⁺ = 307 |
| 12 | 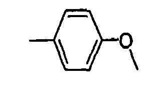 | 6-CI | 5-Cl | H | rac | Cyclohexane 1/ AcOEt 1, Rf=0.27 |
| 13 | 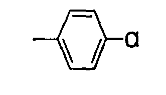 | 6-CI | 5-Me | H | rac | Cyclohexane 1/ AcOEt 1, Rf=0.3 |
| 14 | 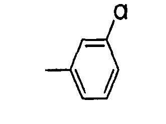 | 6-Cl | 5-Me | H | rac | Cyclohexane 1/ AcOEt 1, Rf=0.3 |
| 15 | 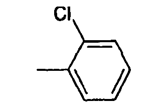 | 6-CF3 | H | H | rac | Cyclohexane 1/ AcOEt 1, Rf=0.3 |
| 16 | 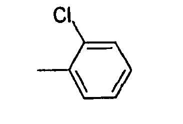 | H | 5-Me | 4-CI | rac | Pf=220°C (M+H)⁺ =307 |
| 17 | 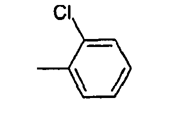 | H | 5-Me | 4-Me | rac | Pf=200°C (M-FH)⁺ =287 |
| 18 | 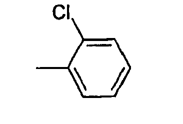 | 6-Cl | H | H | rac | Pf=251°C (M+H)⁺ =293 |
| 19 | 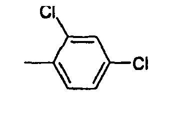 | 6-Cl | 5-Me | H | rac | Pf=255°C M⁻ =339 |
| 20 | 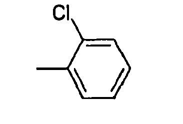 | H | 5-Me | H | rac | Cyclohexane 1/ AcOEt 1, Rf=0.3 |
| 21 | 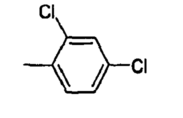 | 7-Br | 6-Cl | 5-Me | rac | Cyclohexane 1/AcOEt 1, Rf=0.3 |
| 22 | 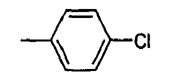 | 6-CI | 5-Me | H | rac | Cyclohexane 1/ AcOEt 1, Rf=0.3 |
| 23 | 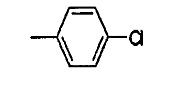 | H | 5-Me | 4-CI | rac | Cyclohexane 1/ AcOEt 1, Rf=0.3 |
| 24 | 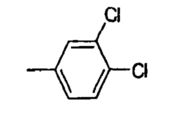 | 6-Cl | 5-Me | H | rac | Cyclohexane 1/ AcOEt 1, Rf=0.3 |
| 25 | 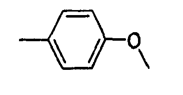 | 6-Cl | 5-Me | H | rac | Cyclohexane 1/ AcOEt 1, Rf=0.27 |
| 26 | 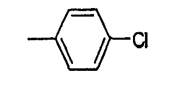 | 6-Me | 5-Cl | H | rac | Cyclohexane 1/ AcOEt 1, Rf=0.3 |
| 27 | 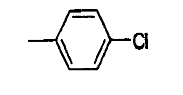 | 6-Cl | 5-Cl | H | rac | (M+H)⁺ =327 |
| 28 | 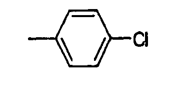 | 6-I | H | 4-CF3 | rac | Cyclohexane 1/AcOEt 1, Rf=0.3 |
| 29 | 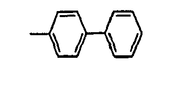 | 6-Cl | 5-Cl | H | rac | Hexane 1/AcOEt 1, Rf=0.45 |
| 30 | 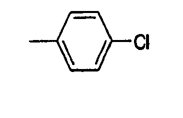 | 6-Cl | 5-Cl | 4-Cl | rac | Pf=246°C M⁻ =359 |
| 31 | 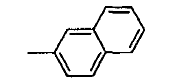 | 6-Cl | 5-Cl | H | rac | Hexanel /AcOEt 1 Rf=0.5 |
| 32 | 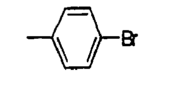 | 6-Cl | 5-Cl | H | rac | (M+H)⁺ =369 |
| 33 | 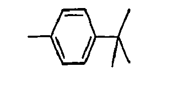 | 6-CI | 5-Cl | H | rac | Hexane 1/ AcOEt 1, Rf=0.45 |
| 34 | 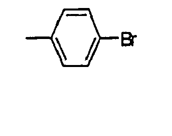 | 6-Cl | 5-Cl | H | (-) | Pf= 240°C Alpha D= -43°, c=0.25% in MeOH |
| 35 | 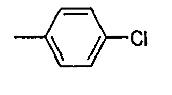 | 7-Cl | 6-H | 5-Cl | rac | Cyclohexane 1/ AcOEt 1, Rf=0.3 |
| 36 | 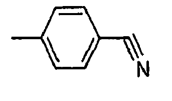 | 6-Cl | 5-Cl | H | rac | Pf=180°C |
| 37 | 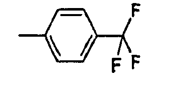 | 6-Cl | 5-Cl | H | rac | Cyclohexane 1/ AcOEt 1, Rf=0.45 |
| 38 | 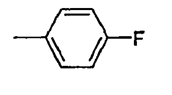 | 6-Cl | 5-Cl | H | rac | Cyclohexane 1/ AcOEt 1, Rf=0.3 |
| 39 | 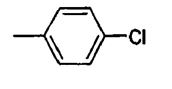 | 6-CN | 5-H | 4-CF3 | rac | M- =350 |
| 40 | 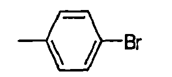 | 6-Cl | 5-H | 4-Cl | rac | Cyclohexane 1/ AcOEt 1, Rf=0.3 |
| 41 | 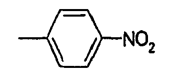 | 6-CI | 5-Cl | H | (+) | Alpha D=+28°, c=0.25% in MeOH |
| 42 | 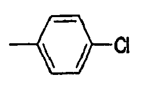 | 6-Cl | H | H | (+) | Alpha D=+19°, C=0.26% in MeOH |
| 43 | 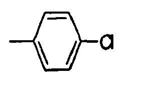 | 6-Cl | H | H | (-) | Alpha D=-23°, c=0.64% in MeOH |
| 44 | 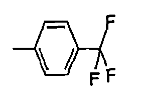 | 6-Cl | 5-H | 4-CI | (+) | Alpha D=+60°, c=0.25% in MeOH |
| 45 | 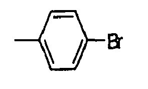 | 6-Cl | 5-CI | H | (+) | Alpha D=+42°, c=0.25% in MeOH |
| 46 | 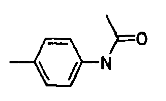 | 6-Cl | 5-Cl | H | (+) | M⁻ =348 |
| 47 | 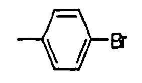 | 6-F | 5-H | 4-F | (+) | Alpha D=+45°, c=0.25% in MeOH M⁻ =339 |
| 48 | 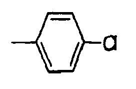 | 6-Cl | 5-H | 4-CI | (+) | Alpha D=+99,2°, c=0.5% in MeOH |
| 49 | 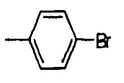 | 6-Cl | 5-H | 4-CI | (+) | Alpha D=+92°, c=0.25% in MeOH |
| 50 | 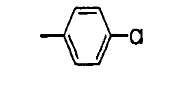 | 6-Ci | 5-Me | H | (-) | Alpha D=-91°, c=0.16% in HCl 3N |
| 51 | 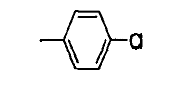 | 6-Cl | 5-Me | H | (+) | Alpha D=+104°, c=0.16% in HCl 3N |
| 52 | 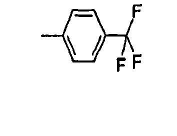 | 6-Cl | 5-Cl | H | (+) | Pf=231 °C Alpha D=+12.4°, c=0.5% in MeOH |
| 53 | 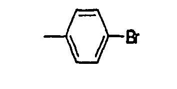 | 6-CF3 | H | 4-Cl | (+) | Alpha D=+88°, c=20% in MeOH |
| 54 | 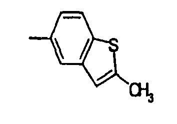 | 6-Cl | H | 4-Cl | (+) | (M+H)⁺ = 363 |
| 55 | 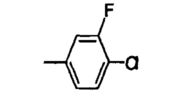 | 6-Cl | H | 4Cl | (+) | Alpha D=+96°, c=0.15% in MeOH |
| 54 | 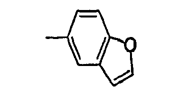 | 6-Cl | H | 4Cl | (+) | Hexane6/AcOEt 4, Rf=0.25 |
| 55 | 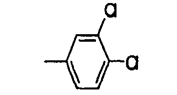 | 6-Cl | H | 4Cl | (+) | (M+H)⁺= 361 |
| 56 | 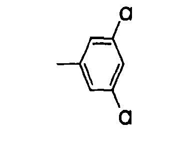 | 6-Cl | H | 4Cl | (+) | Alpha D=+89°, c=0.10% in MeOH |
| 57 | 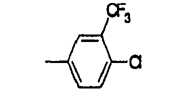 | 6-Cl | H | 4Cl | (+) | Alpha D=+79°, c=0.10% in MeOH |
| 58 | 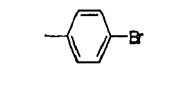 | 6-OMe | H | 4CF3 | (+) | Alpha D=+62°, c=0.50% in MeOH |
| 59 | 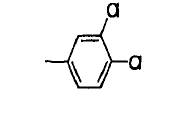 | 6-CI | 5-F | 4Cl | rac | Cyclohexane 7/AcOEt 1, Rf=0.4 |
| 60 | 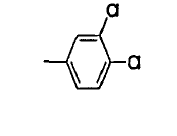 | 6-Cl | H | 4Cl | rac | Cyclohexane 7/AcOEt 1, Rf=0.4 |
| 61 | 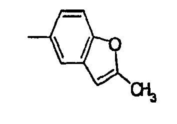 | 6-CI | H | 4Cl | (+) | Alpha D=+62°, c=0.50% in MeOH |
| 62 | 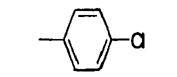 | 6-Br | H | H | (+) | (M-NH₃)⁺ = 320 |
| 63 | 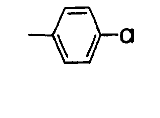 | 6- 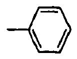 | H | H | (-) | Alpha D=-32°, c=0.17% in MeOH |
| 64 | 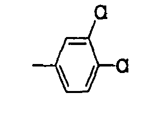 | 6-Cl | H | H | (+) | (M-NH₃)⁺ = 310 |
| 65 | 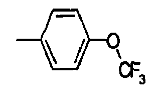 | 6-Cl | H | 4Cl | (+) | (M+H)⁺= 377 |
| 66 | 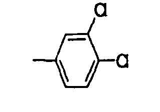 | 6-Br | H | H | rac | Hexane6/AcOEt 4, Rf=0.4 |
| 67 | 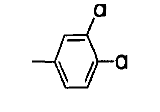 | 6-Br | H | H | (+) | Hexane6/AcOEt 4, Rf=0.4 |
| 68 | 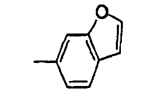 | 6-Cl | H | 4Cl | (+) | Hexane1/AcOEt 1, Rf=0.5 |
| 69 | 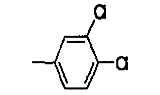 | 6-Cl | H | 4Cl | (+) | (M+H)⁺= 359 |
| 70 | 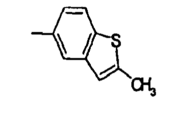 | 6-Cl | H | 4Cl | rac | (M+H)⁺= 363 |
| 71 | 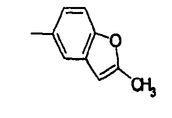 | 6-CI | H | H | (+) | (M+H)⁺ = 313 |
| 72 | 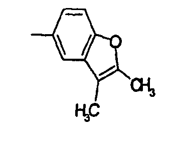 | 6-Cl | H | 4Cl | (+) | (M+H)⁺= 361 |
| 73 | 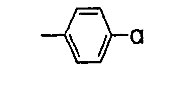 | 5-Br | H | H | (+) | (M+H)⁺ = 337 |
| 74 | 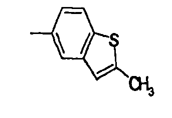 | 6-Br | H | H | (+) | (M-NH₃)⁺= 356 |
| 75 | 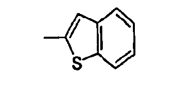 | 6-Cl | H | 4Cl | (+) | Dichloromethane7/ AcOEt 3, Rf=0.85 |
| 76 | 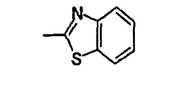 | 6-Cl | H | 4Cl | (+) | Alpha D=+81°, c=0.11 in MeOH |
| 77 | 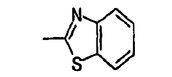 | 6-Cl | H | 4Cl | (-) | Alpha D=-82°, c=0.10 in MeOH |
| 78 | 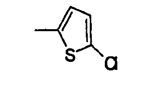 | 6-Cl | H | 4Cl | (+) | Alpha D=+154°, c=0.25 in MeOH |
| 79 | 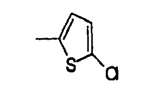 | 6-Cl | H | 4Cl | (-) | Alpha D=-216°, c=0.25 in MeOH |
| 80 | 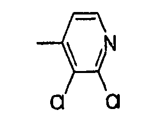 | 6-Cl | H | 4Cl | (-) | Alpha D=-116°, c=0.10 in MeOH |
| 81 | 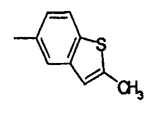 | 6-CI | H | H | (+) | (M+H)⁺ = 327 |
| 82 | 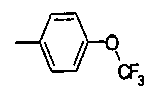 | 6-CI | H | H | (+) | (M-NH₃)⁺= 326 |
| 83 | 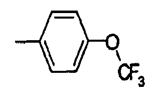 | 6-Br | H | H | (+) | (M+H)⁺= 385 |
| 84 | 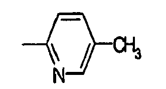 | 6-Cl | H | H | rac | (M+H)⁺ = 274 |

### Exemple 1 :

### (+) N-[5,6-Dichloro-3-(4-chloro-phényl)-2-oxo-2,3-dihydro-1H-indol-3-yl]-2-(4-méthyl-pipérazin-1-yl)-acétamide

### (i) 2-Chloro-N-[5,6-dichloro-3-(4-chloro-phényl)-2-oxo-2,3-dihydro-1H-indol-3-yl]-acétamide :

Dans un ballon muni d'un agitateur magnétique et sous flux d'azote, on charge 1,3 g du produit obtenu à la Préparation 5, 47 ml de toluène, 0,32 ml de pyridine et 0,31 ml de chlorure de chloroacétyle. On laisse réagir à 110°C pendant 4 heures, puis on verse le mélange réactionnel dans l'eau et on extrait à l'acétate d'éthyle. La phase organique est séchée sur Na₂SO₄, filtrée et évaporée sous vide. On obtient 900 mg de solide beige, purifié sur colonne par flash chromatographie au moyen du mélange cyclohexane 8/ acétate d'éthyle 2 pour obtenir 400 mg du produit attendu.
CCM: Hex. 1/AcOEt 1, Rf=0.5

### (ii) (+) N-[5,6-Dichloro-3-(4-chloro-phényl)-2-oxo-2,3-dihydro-1H-indol-3-yl]-2-(4-méthyl-pipérazin-1-yl)-acétamide:

Dans un ballon muni d'un agitateur magnétique, on charge 0,4 g du produit de l'étape précédente, 0,11 ml de N-méthylpipérazine (d 0,903), 0,14 g de carbonate de potassium, 0,07 g de iodure de sodium dans 8 ml de DMF. On laisse réagir à 60°C pendant 4 heures puis on verse le mélange réactionnel dans l'eau et on extrait à l'acétate d'éthyle. La phase organique est séchée sur Na₂SO₄, filtrée et évaporée sous vide. On obtient 240 mg d'huile que l'on reprend à l'éther éthylique pour obtenir 140 mg de solide blanc. Les eaux de filtration sont purifiées par flash chromatographie au moyen de l'éluant acétate d'éthyle 9 / méthanol 1, puis acétate d'éthyle 7 / méthanol 3, de façon à isoler 40 mg de solide blanc.
P.f. = 207.1-207.6°C ; [α_{D}]= +141°, c=0.25 wt% MeOH ; ¹H NMR δ (ppm, dmso-d6): 2.37 (sb, 3H), 2.50 - 2.74 (m, **), 2.94 - 3.16 (m, 2H), 7.08 (s, 1H), 7.31 (m, 2H), 7.48 (m, 2H), 7.52 (s, 1H), 8.71 (s, 1H), 10.70 (s, 1H). LCMS: (M+H)⁺ = m/z 467 uma

### Exemple 2:

### (+) N-[4,6-Dichloro-3-(4-trifluorométhyl-phényl)-2-oxo-2,3-dihydro-1H-indol-3-yl]-2-(1-ethyl-piperidin-4-yl)-acétamide

1) Sous flux d'azote, on ajoute dans 4,4 ml de dichlorométhane anhydre refroidi dans un bain de glace, 172,84 mg de PCl₅ puis on ajoute lentement 142,13 mg de l'acide de la Préparation 2. On laisse agir le mélange réactionnel à 0°C pendant 10 minutes puis à température ambiante pendant 3 heures.
2) D'autre part, on suspend sous flux d'azote 100 mg du produit de la preparation 8, dans 4,4 ml de dichlorométhane puis on ajoute 0,1 ml de pyridine. On refroidit dans un bain de glace. On ajoute goutte à goutte la solution préparée en 1) et on agite à température ambiante pendant une heure.

On verse le mélange réactionnel dans l'eau et on extrait à l'acétate d'éthyle. La phase organique est lavée avec une solution saturée de NaHCO₃, séchée sur Na₂SO₄, filtrée et évaporée sous vide. On obtient 145 mg de solide orange, purifié sur colonne par flash chromatographie au moyen de l'éluant acétate d'éthyle 1 / méthanol 1 pour obtenir 85 mg de produit repris à l'isopropyléther de façon à obtenir 75 mg de produit solide blanc/rosé.
P.f. =158-162°C ; [α_{D}]= +194°, c=0.125 wt% in MeOH, ; RMN ¹H : δ (ppm, dmso-d6): 0.97 (t, J= 7.1 Hz, 3H), 1.07 - 1.23 (m, 2H), 1.47 - 1.67 (m, 3H), 1.71 - 1.85 (m, 2H), 2.07 - 2.22 (m, 2H), 2.27 (q, J= 7.1 Hz, 2H), 2.78 (m, 2H), 6.90 (sb, 1H), 7.18 (sb, 1H), 7.47 (m, 2H), 7.78 (m, 2H), 9.15 (s, 1H), 10.74 (sb, 1H); LCMS: (M+H)⁺ = m/z 514 uma

### Exemple 3 :

### N-[4,6-Dichloro-3-(4-chloro-phényl)-2-oxo-2,3-dihydro-1H-indol-3-yl]-2-(4-méthyl-pipérazin-1-yl)-acétamide

En opérant comme décrit dans l'exemple 2 mais en utilisant le composé de la Préparation 4 au lieu du composé de la Préparation 2 et le composé de la Préparation 7 au lieu du composé de la Préparation 5 on obtient le composé titre.
P.f. =248-251°C, ;LCMS: (M+H)⁺= m/z 467 uma ; RMN ¹H : δ (ppm, dmso-d6): 2.16 (s, 3H), 2.24 - 2.42 (m, 4H), 2.43 - 2.57 (m, **), 2.98 - 3.12 (m, 2H), 6.92 (d, J= 1.8Hz, 1H), 7.18 (d, J= 1.8Hz, 1H), 7.26 (m, 2H), 7.50 (m, 2H), 8.70 (s, 1H), 10.78 (s, 1H

### Exemple 4 :

### N-[4-trifluoromethyl-6-cyano-3-(4-chloro-phényl)-2-oxo-2,3-dihydro-1H-indol-3-yl]-2-(4-éthyl-pipérazin-1-yl)-acétamide

En opérant comme décrit dans l'exemple 1 mais en utilisant le produit de la Préparation 28 au lieu du produit de la Préparation 5 et la N-éthylpipérazine au lieu de la N-méthylpipérazine on obtient le composé titre.
P.f. = 260-262°C; LCMS: (M+H)⁺ = m/z 506 uma; RMN ¹H: δ (ppm, dmso-d6): 0.98 (t, J= 7.3Hz, 3H), 2.23 - 2.42 (m, 6H), 2.42 - 2.61 (m, **), 2.87 - 3.17 (m, 2H), 7.14 (m, 2H), 7.47 (m, 2H), 7.59 (sb, 1H), 7.91 (sb, 1H), 8.91 (s, 1H), 11.12 (sb, 1H).

### Exemple 5:

### (+) N-[1-Benzoyl-5,6-Dichloro-3-(4-chloro-phényl)-2-oxo-2,3-dihydro-1H-indol-3-yl]-2-(4-méthyl-pipérazin-1-yl)-acétamide

Dans un ballon muni d'un agitateur magnétique et sous flux d'azote, on charge 0,14 g du composé de l'exemple 1 dans 9 ml de DMF. A 0°C, on ajoute 0,01 g de NaH à 60%. Puis on verse goutte à goutte PhCOCl et on laisse agiter à température ambiante pendant 2h. On verse le mélange réactionnel dans l'eau et on extrait à l'acétate d'éthyle. La phase organique est séchée sur Na₂SO₄, filtrée et évaporée sous vide. On obtient 120 mg d'huile, que l'on purifie sur colonne par flash chromatographie au moyen de l'éluant acétate d'éthyle 95 / méthanol 5, de façon à isoler 20 mg de solide blanc.
P.f. =92-94°C; ¹H RMN: δ (ppm, dmso-d6): 2.16 (s, 3H), 2.24 - 2.40 (m, 4H), 2.40 - 2.50 (m, **), 2.94 - 3.16 (m, 2H), 7.30 (m, 2H), 7.45 (m, 2H), 7.54 (m, 2H), 7.58 - 7.66 (m, 3H), 7.74 (s, 1 H), 8.00 (s, 1H), 9.14 (s, 1H); LCMS: (M+H)⁺ = m/z 571 uma

### Exemple 6 :

### 3-(4-Chloro-phényl)-3-[2-(4-ethyl-pipérazin-1-yl)-acétylamino]-2-oxo-4-trifluorométhyl-2,3-dihydro-1H-indole-6-carboxamide

Dans un ballon muni d'un agitateur magnétique, on charge 0,16 g du produit obtenu à l'exemple 4, 0,47 g de potasse et 7 ml de tBu-OH. On laisse réagir à 50°C pendant 5 heures. On filtre sur célite, on lave avec du THF. On évapore le filtrat sous vide que l'on reprend avec de l'acétate d'éthyle et qu'on lave avec de l'eau. La phase organique est séchée sur Na₂SO₄, filtrée et évaporée sous vide. On isole 100 mg d'une huile, qui est purifiée par flash chromatographie avec l'éluant acétate d'éthyle 8/ méthanol 2. On isole 10 mg de solide blanc.
¹H RMN: δ (ppm, dmso-d6): 0.99 (t, J= 7.2Hz, 3H), 2.25 - 2.42 (m, 6H), 2.42 - 2.62 (m, **), 2.92 - 3.16 (m, 2H), 7.12 (m, 2H), 7.47 (m, 2H), 7.55 (sb, 1H), 7.64 (sb, 1H), 7.84 (sb, 1H), 8.24 (sb, 1H), 8.75 (s, 1H), 10.95 (sb, 1H) ; LCMS : (M+H)⁺ = m/z 571 uma

### Exemple 7

### N-[6-Chloro-3-(4-chloro-phényl)-1,5-diméthyl-2-oxo-2,3-dihydro-1H-indol-3-yl]-2-(4-méthyl-pipérazin-1-yl)-acétamide

En opérant comme décrit dans l'exemple 1 mais en utilisant le produit de la Préparation 9 au lieu du produit de la Préparation 5, on obtient le composé titre.
P.f. = 217-219°C ; RMN: ¹H : δ (ppm, dmso-d6): 2.26 - 2.35 (m, 6H), 2.44 - 2.64 (m, **), 2.89 - 3.15 (m, 5H), 7.19 (s, 1H), 7.25 - 7.33 (m, 3H), 7.46 (m, 2H), 8.71 (sb, 1H). LCMS: (M+H)⁺ = m/z 461 uma

### Exemple 8

### (+) N-[4,6-Dichloro-3-(4-trifluorométhyl-phényl)-2-oxo-2,3-dihydro-1H-indol-3-yl]-2-(1-ethyl-1,2,3,6-tetrahydro-pyrid-4-yl)-acétamide

En opérant comme décrit dans l'exemple 2 mais en utilisant le produit de la Préparation 10 au lieu du produit de la Préparation 2, on obtient le composé titre.

P.f. = 155-156°C ; RMN ¹H : δ (ppm, dmso-d6): 1.03 (t, J= 7.2Hz, 3H), 2.06 (m, 2H), 2.31 - 2.65 (m, **), 2.80 - 3.05 (m, 4H), 5.46 (sb, 1H), 6.91 (d, J= 1.7Hz, 1H), 7.20 (d, J= 1.7Hz, 1H), 7.49 (m, 2H), 7.79 (m, 2H), 9.3 (s, 1H), 10.8 (s, 1H).
LCMS: (M+H)⁺ = m/z 459uma

Le tableau 2 qui suit illustre les structures chimiques et les propriétés physiques de quelques exemples de composés selon l'invention. Dans ce tableau :
- dans la colonne « stéréoisomère », « rac » représente un mélange racémique, et (+) ou (-) représente l'un ou l'autre des stéréoisomères,
- dans la colonne « sel », « - » représente un composé sous forme de base libre, alors que « HCl » représente un composé sous forme de chlorhydrate et le rapport entre parenthèses est le rapport (acide:base),
- Me, Et, n-Pr, i-Pr, n-Bu et i-Bu représentent respectivement des groupes méthyle, éthyle, n-propyle, isopropyle, n-butyle et isobutyle, et
- Ph et Bn représentent respectivement des groupes phényle et benzyle.

**TABLEAU 2**

| | | | | | | | | | | | | |
|---|---|---|---|---|---|---|---|---|---|---|---|---|
| 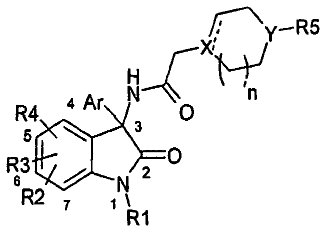 | | | | | | | | | | | | |

| Ex. N° | 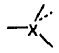 | Y | Ar | R₁ | R₂ | R₃ | R₄ | R₅ | n | sel | Isomère | Alpha D, LCMS ou P.F. |
|---|---|---|---|---|---|---|---|---|---|---|---|---|
| 1 | -N< | >N- | 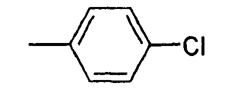 | H | 6-Cl | 5-Cl | H | Me | 1 | - | (+) | 167-168°C |
| 2 | -CH< | >N- | 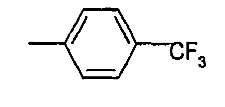 | H | 6-Cl | H | 4-Cl | Et | 1 | - | (+) | 158-162°C |
| 3 | -N< | >N- | 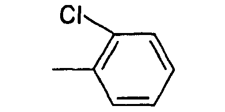 | H | 6-Cl | 5-Me | H | Me | 1 | - | rac | 256-258°C |
| 4 | -N< | >N- | 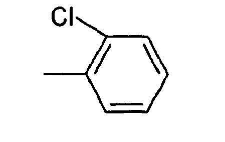 | H | 6-CI | 5-Me | H | Me | 2 | - | rac | 130°C |
| 5 | -N< | >N- | 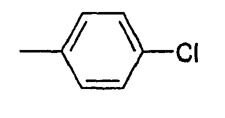 | H | 6-Cl | 5-Me | H | Me | 1 | - | rac | m/z= 447 |
| 6 | -N< | >N- | 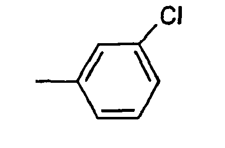 | H | 6-Cl | 5-Me | H | Me | 1 | - | rac | m/z= 447 |
| 7 | -N< | >N- | 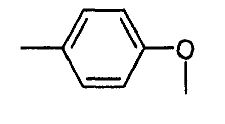 | H | 6-Cl | 5-Cl | H | Me | 1 | - | rac | 263°C |
| 8 | 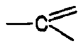 | >N- | 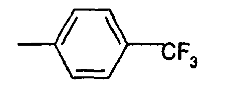 | H | 6-Cl | H | 4-Cl | Et | | - | (+) | m/z= 512 |
| 9 | -N< | >N- | 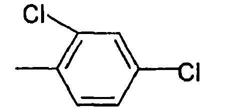 | H | 6-Cl | 5-Me | H | Me | 1 | - | rac | 144°C |
| 10 | -N< | >N- | 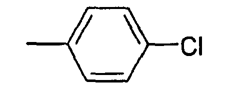 | H | 6-Cl | 5-Me | H | Me | 1 | - | (+) | 152-153°C |
| 11 | -N< | >N- | 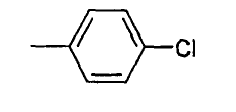 | H | 6-Cl | 5-Me | H | Me | 1 | - | (-) | 185°C |
| 12 | -N< | >N- | 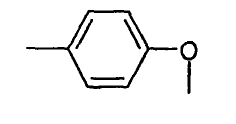 | H | 6-Cl | 5-Me | H | Me | 1 | - | rac | 256°C |
| 13 | -N< | >N- | 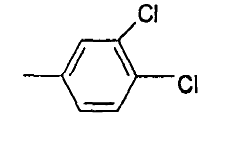 | H | H | 5-Me | 4-Cl | Me | 1 | - | rac | 282-284°C |
| 14 | -N< | >N- | 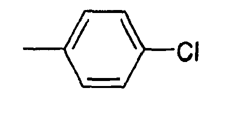 | H | 6-Cl . | 5-Me | H | Me | 1 | - | rac | 229-232°C |
| 15 | -N< | >N- | 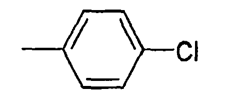 | H | 6-Me | 5-Cl | H | Me | 1 | - | rac | 267-269°C |
| 16 | -N< | >N- | 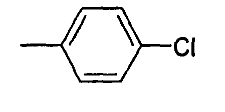 | H | 6-Cl | 5-Me | H | Et | 1 | - | rac | 223-224°C |
| 17 | -N< | >N- | 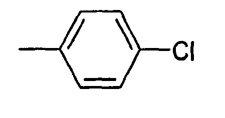 | H | 6-Cl | 5-Cl | H | Me | 2 | - | rac | 214-216°C |
| 18 | -N< | >N- | 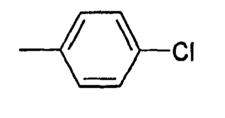 | H | 6-Cl | 5-Cl | H | Me | 1 | - | (-) | 188-190°C |
| 19 | -N< | >N- | 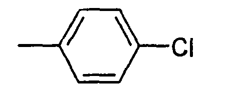 | H | 6-Cl | 5-Cl | 4-Cl | Me | 1 | - | rac | 276-278°C |
| 20 | -N< | >N- | 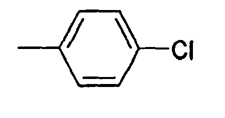 | H | 6-I | H | 4-CF₃ | Me | 1 | - | rac | 231-232°C |
| 21 | -N< | >N- | 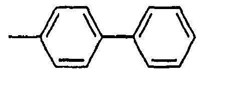 | H | 6-Cl | 5-Cl | H | Me | 1 | - | rac | 181-182°C |
| 22 | -N< | >N- | 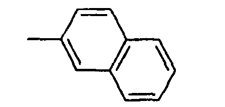 | H | 6-Cl | 5-Cl | H | Me | 1 | - | rac | 237-238°C |
| 23 | -N< | >N- | 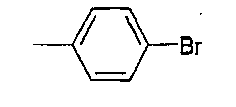 | H | 6-Cl | 5-Cl | H | Me | 1 | - | rac | 291-294°C |
| 24 | -N< | >N- | 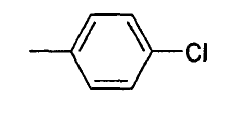 | H | 6-Cl | 5-Cl | H | Me | 1 | - | rac | 293-294°C |
| 25 | -N< | >N- | 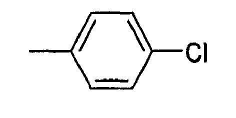 | H | 6-Cl | 5-Cl | H | Et | 1 | - | rac | 227-229°C |
| 26 | -N< | >CH- | 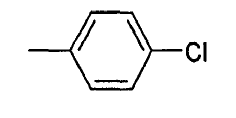 | H | 6-Cl | 5-Cl | H | Me | 1 | - | rac | 261-262°C |
| 27 | -N< | >N- | 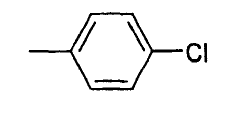 | H | 6-Cl | H | 4-Cl | Me | 1 | - | rac | 248-251°C |
| 28 | -N< | >N- | 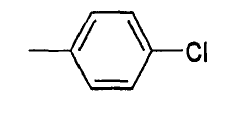 | H | 7-Cl | 5-Cl | H | Me | 1 | - | rac | 260-261°C |
| 29 | -N< | >N- | 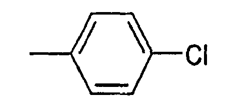 | H | 6-Cl | 5-Cl | H | Et | 2 | - | rac | 189-190°C |
| 30 | -N< | >N- | 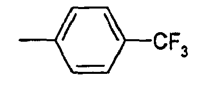 | H | 6-Cl | 5-Cl | H | Me | 1 | - | rac | 267-269°C |
| 31 | -N< | >N- | 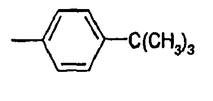 | H | 6-Cl | 5-Cl | H | Me | 1 | - | rac | 207-209°C |
| 32 | -N< | >N- | 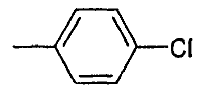 | H | 6-Cl | 5-Me | H | Et | 1 | - | (+) | 158-159°C |
| 33 | -N< | >N- | 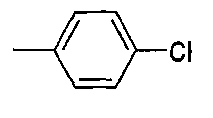 | H | 6-Cl | 5-Cl | H | Et | 1 | - | (+) | 160-163°C |
| 34 | -N< | >N- | 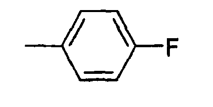 | H | 6-Cl | 5-Cl | H | Me | 1 | - | rac | 282-284°C |
| 35 | -N< | >N- | 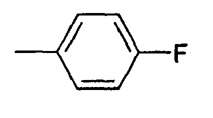 | H | 6-Cl | 5-Cl | H | Et | 1 | - | rac | 274-275°C |
| 36 | -N< | >N- | 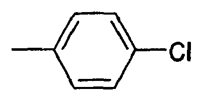 | H | 6-Cl | H | 4-Cl | Me | 1 | - | (+) | 157-158°C |
| 37 | -N< | >N- | 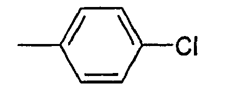 | H | 6-Cl | H | 4-Cl | Et | 1 | - | (+) | 155°C |
| 38 | -N< | >N- | 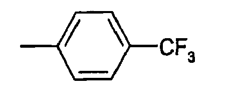 | H | 6-Cl | 5-Cl | H | Me | 1 | - | (+) | 255-256°C |
| 39 | -N< | >N- | 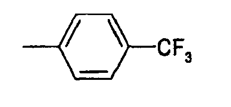 | H | 6-Cl | 5-Cl | H | CH(Me)₂ | 1 | - | (+) | 221°C |
| 40 | -N< | >N- | 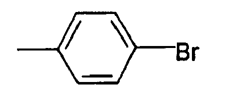 | H | 6-Cl | 5-Cl | H | Me | 1 | - | (+) | 292-294°C |
| 41 | -N< | >N- | 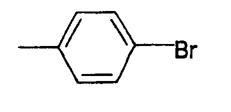 | H | 6-Cl | 5-Cl | H | Et | 1 | - | (+) | 198-201°C |
| 42 | -N< | >N- | 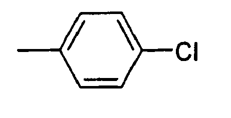 | H | 6-Cl | 5-Cl | H | Me | 2 | - | (+) | 214-216°C |
| 43 | -N< | >N- | 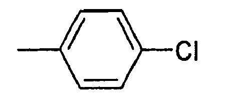 | 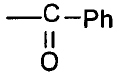 | 6-Cl | 5-Cl | H | Me | 1 | - | (+) | 92-94°C |
| 44 | -N< | >N- | 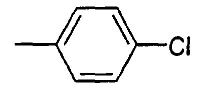 | 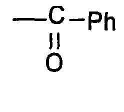 | 6-Cl | 5-Cl | H | Me | 1 | - | rac | 198°C |
| 45 | -N< | >N- | 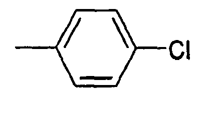 | 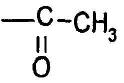 | 6-Cl | 5-Cl | H | Me | 1 | - | rac | 225°C |
| 46 | -N< | >N- | 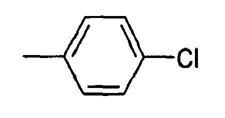 | -Me | 6-Cl | 5-Me | H | Me | 1 | - | rac | 217-219°C |
| 47 | -N< | >N- | 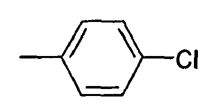 | 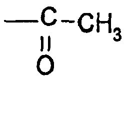 | 6-Cl | 5-Cl | H | Et | 1 | - | rac | 93-94°C |
| 48 | -N< | >N- | 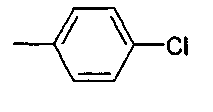 | 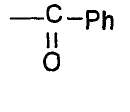 | 6-Cl | 5-Cl | H | Et | 1 | - | rac | 194-195°C |
| 49 | -N< | >N- | 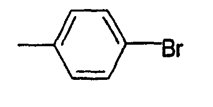 | 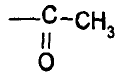 | 6-Cl | 5-Cl | H | Me | 1 | - | rac | 223-224°C |
| 50 | -N< | >N- | 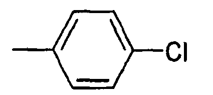 | H | 6-Cl | 5-Cl | H | Me | 1 | - | rac | 293-294°C |
| 51 | -CH< | >N- | 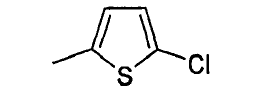 | H | 4-Cl | 6-Cl | H | Et | 1 | - | (+) | m/z= 486 |
| 52 | -CH< | >N- | 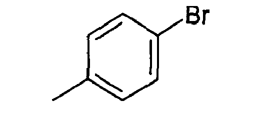 | H | 4-Cl | 6-CF3 | H | Et | 1 | - | (+) | m/z= 558 |
| 53 | -N< | >N- | 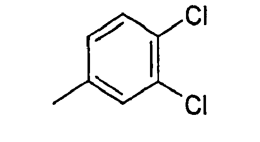 | H | 4-Cl | 6-Cl | H | Et | 1 | - | (+) | m/z= 515 |
| 54 | -CH< | >N- | 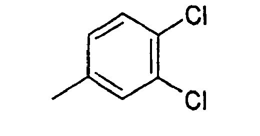 | H | 4-Cl | 6-Cl | H | Et | 1 | - | (+) | 211-213°C |
| 55 | -CH< | >N- | 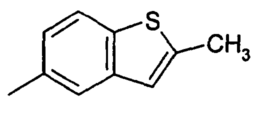 | H | 4-Cl | 6-Cl | H | Et | 1 | - | (+) | m/z= 516 |
| 56 | -CH< | >N- | 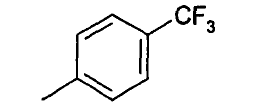 | 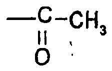 | 4-Cl | 6-Cl | H | Et | 1 | - | (+) | 162-163°C |
| 57 | -CH< | >N- | 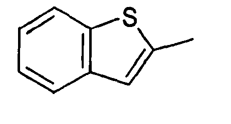 | H | 4-Cl | 6-Cl | H | Et | 1 | - | (+) | m/z= 502 |
| 58 | -CH< | >N- | 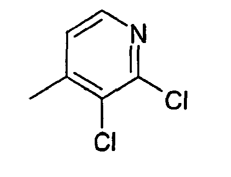 | H | 4-Cl | 6-Cl | H | Et | 1 | - | (+) | 177°C |
| 59 | -CH< | >N- | 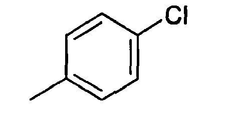 | -Me | 4-Cl | 6-Cl | H | Et | 1 | - | rac | 293°C |
| 60 | -CH< | >N- | 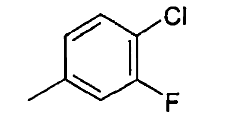 | H | 4-Cl | 6-Cl | H | Et | 1 | - | (+) | 196-200°C |
| 61 | -N< | >N- | 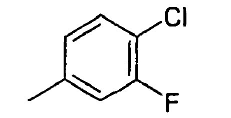 | H | 4-Cl | 6-Cl | H | Et | 1 | - | (+) | m/z= 499 |
| 62 | -N< | >N- | 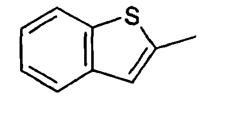 | H | 4-Cl | 6-Cl | H | Et | 1 | - | (+) | m/z= 503 |
| 63 | -CH< | >N- | 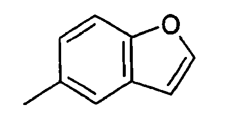 | H | 4-Cl | 6-Cl | H | Et | 1 | - | (+) | 163-166°C |
| 64 | -N< | >N- | 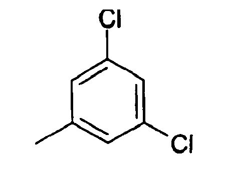 | H | 4-Cl | 6-Cl | H | Et | 1 | - | (+) | 140-142°C |
| 65 | -N< | >N- | 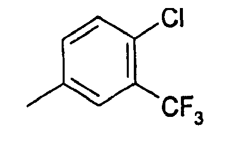 | H | 4-Cl | 6-Cl | H | Et | 1 | HCl | (+) | 150-156°C |
| 66 | -N< | >N- | 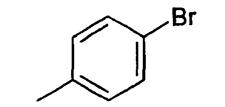 | H | 4-CF3 | 6-OMe | H | Et | 1 | - | (+) | m/z= 555 |
| 67 | -CH< | >N- | 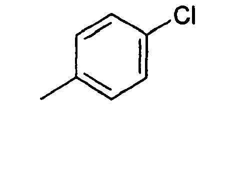 | -Me | 4-Cl | 6-Cl | H | Et | 1 | - | (-) | Alpha D= -205°, c= 0.135 in MeOH |
| 68 | -CH< | >N- | 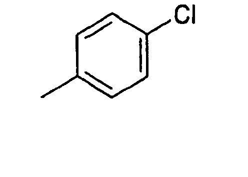 | -Me | 4-Cl | 6-Cl | H | Et | 1 | - | (+) | Alpha D= +145°, c= 0.28 in MeOH |
| 69 | -N< | >N- | 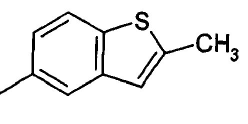 | H | 4-Cl | 6-Cl | H | Et | 1 | - | (+) | 180-182°C |
| 70 | -N< | >N- | 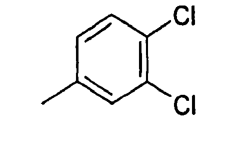 | H | 4-Cl | 6-Cl | 5-F | Et | 1 | - | (+) | 148-150°C |
| 71 | -CH< | >N- | 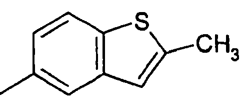 | i-Pr | 4-Cl | 6-Cl | H | Et | 1 | - | rac. | 163-165°C |
| 72 | -CH< | >N- | 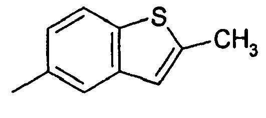 | Et | 4-Cl | 6-Cl | H | Et | 1 | - | rac. | 154-156°C |
| 73 | -N< | >N- | 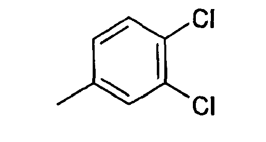 | H | 4-Cl | 6-Cl | H | 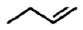 | 1 | - | (+) | m/z= 527 |
| 74 | -CH< | >N- | 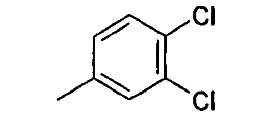 | i-Pr | 4-Cl | 6-Cl | H | Et | 1 | - | (+) | 115-116°C |
| 75 | -N< | >N- | 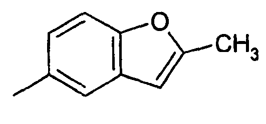 | H | 4-Cl | 6-Cl | H | Et | 1 | - | (+) | 140-142°C |
| 76 | -CH< | >N- | 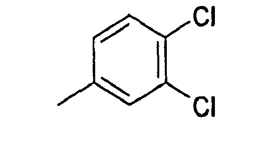 | Et | 4-Cl | 6-Cl | H | Et | 1 | HCl | (-) | 218-220°C |
| 77 | -CH< | >N- | 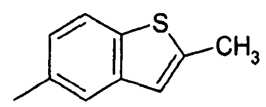 | i-Pr | 4-Cl | 6-Cl | H | Et | 1 | - | (+) | Alpha D= -244°, c= 0.11 in MeOH |
| 78 | -CH< | >N- | 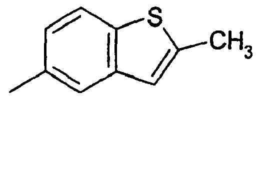 | i-Pr | 4-Cl | 6-Cl | H | Et | 1 | - | (-) | Alpha D= -226°, c= 0.10 in MeOH |
| 79 | -CH< | >N- | 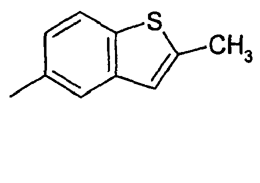 | Et | 4-Cl | 6-Cl | H | Et | 1 | - | (+) | Alpha D= +242°, c=0.11 in MeOH |
| 80 | -CH< | >N- | 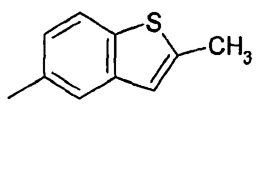 | Et | 4-Cl | 6-Cl | H | Et | 1 | - | (-) | Alpha D= -250°, c= 0.10 in MeOH |
| 81 | -N< | >N- | 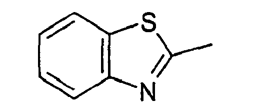 | H | 4-Cl | 6-Cl | H | Et | 1 | - | (+) | 147-148°C |
| 82 | -N< | >N- | 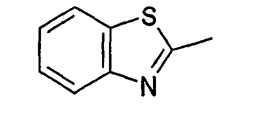 | H | 4-Cl | 6-Cl | H | Et | 1 | - | (-) | m/z= 504 |
| 83 | -N< | >N- | 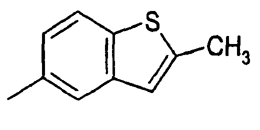 | H | 4-Cl | 6-Cl | H | Me | 1 | - | (+) | m/z= 503 |
| 84 | -N< | >N- | 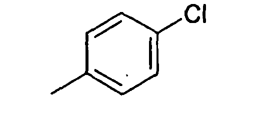 | H | H | 6-Br | H | Et | 1 | - | (+) | 176-178°C |
| 85 | -N< | >N- | 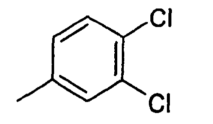 | H | 4-Cl | 6-Cl | H | Me | 2 | Oxalate | (+) | 176-180°C |
| 86 | -N< | >N- | 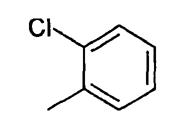 | 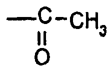 | 5-Me | 6-Cl | H | Et | 1 | Oxalate | (+) | 137-141°C |
| 87 | -N< | >N- | 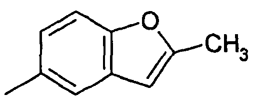 | H | 4-Cl | 6-Cl | H | Me | 1 | Oxalate | (+) | 202-204°C |
| 88 | -N< | >N- | 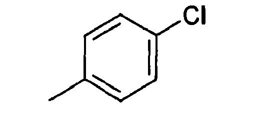 | H | H | 6-Br | H | Me | 1 | - | (+) | 186-187°C |
| 89 | -N< | >N- | 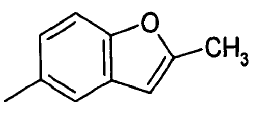 | H | H | 6-Cl | H | Et | 1 | - | (+) | 168°C |
| 90 | -N< | >N- | 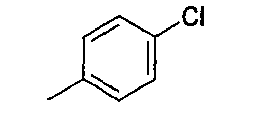 | H | H | 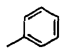 6- | H | Et | 1 | - | (+) | 350°C |
| 91 | -N< | >N- | 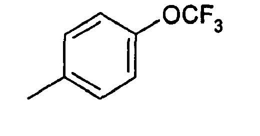 | H | 4-Cl | 6-Cl | H | Et | 1 | - | (+) | 135-137°C |
| 92 | -N< | >N- | 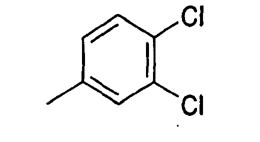 | H | H | 6-Cl | H | Et | 1 | - | (+) | 197°C |
| 93 | -N< | >N- | 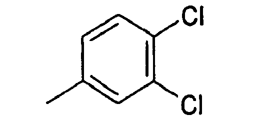 | H | H | 6-Cl | H | Me | 1 | - | (+) | 176°C |
| 94 | -N< | >N- | 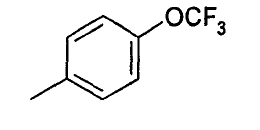 | H | H | 6-Br | H | Et | 1 | Oxalaté | (+) | 161°C |
| 95 | -N< | >N- | 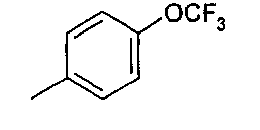 | H | H | 6-Cl | H | Et | 1 | - | (+) | m/z =497 |
| 96 | -N< | >N- | 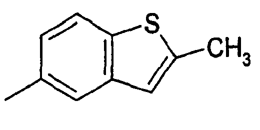 | H | 4-Cl | 6-Cl | H | Me | 2 | Oxalate | rac | 205°C |
| 97 | -N< | >N- | 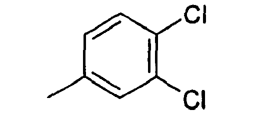 | H | H | 6-Br | H | Et | 1 | Oxalate | (+) | 190°C |
| 98 | -N< | >N- | 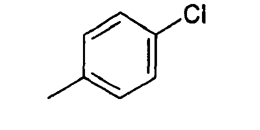 | H | H | 5-Br | H | Et | 1 | - | (+) | 123°C |
| 99 | -N< | >N- | 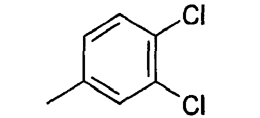 | H | H | 6-Br | H | Me | 1 | Oxalate | (+) | 188°C |
| 10 0 | -N< | >N- | 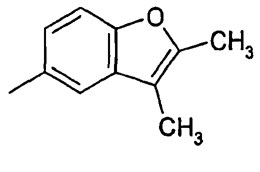 | H | 4-Cl | 6-Cl | H | Et | 1 | - | (+) | 195°C |
| 10 1 | -N< | >N- | 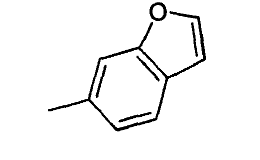 | H | 4-Cl | 6-Cl | H | Et | 1 | Oxalate | (+) | 167°C |
| 10 2 | -N< | >N- | 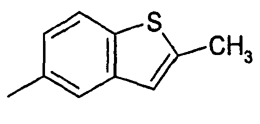 | H | H | 6-Br | H | Et | 1 | Oxalate | (+) | 189°C |
| 10 3 | -N< | >N- | 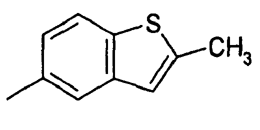 | H | H | 6-Cl | H | Et | 1 | - | (+) | m/z =483 |
| 10 4 | -N< | >N- | 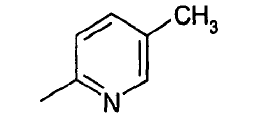 | H | H | 6-Cl | H | Et | 1 | Oxalate | rac | m/z =428 |

### Exemple 53:

RMN ¹H : δ (ppm, dmso-d6): 0.86-1.15 (m; 3H); 2.52-2.78 (m; **); 3.02-3.19 (m; *); 6.93 (d; J= 1.5 Hz; 1H); 7.06 (dd; Ja= 8 Hz; Jb= 2 Hz; 1H); 7.21 (d; J= 1.5 Hz; 1H); 7.56 (d; J= 2 Hz; 1H); 7.67 (d; J= 8 Hz; 1H); 8.96 (bs; 1H); 10.88 (s; 1H).

### Exemple 61:

RMN ¹H : δ (ppm, dmso-d6): 1.08-1.37 (m; 3H); 2.55-3.50 (m; ** ; *); 6.94 (d; J= 2 Hz; 1H); 6.98 (dd; Ja= 8 Hz; Jb= 2 Hz; 1H); 7.21 (d; J= 2 Hz; 1H); 7.33 (dd; Ja= 10 Hz; Jb= 2 Hz; 1H); 7.63 (m; 1H); 8.99 (s; 1H); 9.83 (bs; 1H) ; 10.88 (s; 1H).

### Exemple 65:

RMN ¹H : δ (ppm, dmso-d6): 1.14-1.28 (m; 3H); 2.55-2.80 (m; 2H); 2.80-3.74 (m; *); 6.96 (d; J= 2 Hz; 1H); 7.24 (d; J= 2 Hz; 1H); 7.36 (dd; Ja= 8 Hz; Jb= 2 Hz; 1H); 7.75 (d; J= 8 Hz; 1H); 7.84 (d; J= 2 Hz; 1H); 9.27 (s; 1H); 9.90 (bs; 1H) ; 10.96 (s; 1H).

### Exemple 72:

RMN ¹H : δ (ppm, dmso-d6): 0.96-1.14 (m; 6H); 1.14-1.46 (m; 4H); 1.49-1.63 (m; 1H); 1.63-1.83 (m; 2 H); 2.16 (dd; Ja= 13 Hz ; Jb= 7 Hz; 1H); 2.23 (dd; Ja= 13 Hz ; Jb= 7 Hz; 1H); 2.55 (s; 3H); 2.81-3.12 (m; 2H); 3.65 (m→q; J= 7 Hz; 2H); 7.08-7.19 (m; 2H); 7.22 (d; J= 1.8 Hz; 1H); 7.30 (d; J= 1.8 Hz ; 1H); 7.57 (d; J= 2Hz; 1H); 7.86 (d; J= 9 Hz; 1H); 9.16 (s; 1H).

### Exemple 75:

RMN ¹H : δ (ppm, dmso-d6): 0.98 (t; J= 7 Hz; 3H); 2.29 (m→q; J= 7 Hz; 2H); 2.33-2.41 (m; 4H); 2.44 (s; 3H); 2.52-2.63 (m; **); 3.01 (d; J= 15 Hz; 1H); 3.07 (d; J= 15Hz; 1 H); 6.61 (s; 1H); 6.91 (d; J= 2 Hz; 1H); 7.11 (dd; Ja= 8 Hz; Jb= 2 Hz; 1H); 7.18 (d; J= 2 Hz ; 1H); 7.37 (d; J= 2 Hz; 1H); 7.52 (d; J= 8 Hz; 1H); 8.60 (s; 1H); 10.69 (bs ; 1H).

### Exemple 79:

RMN ¹H : δ (ppm, dmso-d6): 0.98 (t; J= 7 Hz; 3H); 2.30 (m→q; J= 7 Hz; 2 H); 2.33-2.58 (m; **); 3.02 (d; J= 15 Hz; 1H); 3.08 (d; J= 15Hz; 1 H); 6.92 (d; J= 1.6 Hz; 1H); 7.20 (d; J= 1.6 Hz; 1 H); 7.33-7.41 (m; 2H); 7.41-7.47 (m; 2H); 8.78 (bs; 1H); 10.79 (bs ; 1H).

### Exemple 91:

RMN ¹H : δ (ppm, dmso-d6): 0.99 (t; J= 7 Hz; 3H); 1.07 (t; J= 7 Hz; 3H); 1.12-1.29 (m; 2H); 1.47-1.57 (m; 1H); 1.57-1.72 (m; 2H); 1.81-1.98 (m; 2H); 2.12 (dd; Ja= 13 Hz; Jb= 7 Hz; 1 H); 2.22 (dd; Ja= 13 Hz; Jb= 7 Hz; 1H); 2.26-2.40 (m; 2 H); 2.55 (s ; 3H); 2.76-2.91 (m; 2H); 3.64 (m→q; J= 7Hz; 2H); 7.07-7.18 (m; 2H); 7.22 (bs; 1H); 7.29 (bs; 1H); 7.57 (bs; 1H); 7.83 (d; J= 9 Hz; 1H); 9.13 (bs; 1H).

Les composés selon l'invention ont fait l'objet d'études *in vivo.*

### Essai in vivo

Des rats mâles Crl CD BR (Charles River, Italie) pesant 150-175 g ont été logés dans une chambre à température (22±1°C), humidité (55 ± 10%) contrôlées et un cycle lumière-obscurité de 12 heures pendant au moins 7 jours avant leur utilisation. La nourriture et l'eau étaient disponibles *ad libitum.* L'alimentation a été retirée 18 heures avant le sacrifice des animaux. Les rats ont été sacrifiés par dislocation cervicale, l'estomac a été retiré chirurgicalement, ouvert le long de la courbure la plus faible, et placés dans une solution de Krebs (de composition (mM) : NaCl 118, 4; KCI 4,7 ; CaCl₂ 2,5 ; NaH₂PO₄ 3,7 ; MgSO₄ 1,2 ; NaHCO₃ 25 ; glucose 5,6). Le soin et le sacrifice des animaux ont été effectués selon le code éthique international de Sanofi-Aventis et les principes internationaux régissant le soin et le traitement des animaux de laboratoire (E.E.C. Directive 86/609, DJL358, 1, 12 décembre 1987). Des bandes d'environ 1 cm (5 mm de largeur) de fundus gastrique ont été coupées le long de l'axe longitudinal et suspendus dans 20 ml de bain rempli avec la solution de Krebs à 37°C et gazés avec un mélange gazeux de 95% O₂-5% CO₂. Les bandes ont été maintenues à une charge de repos de 1 g et après lavage de choline (précurseur de l'acétylcholine) 10 µM et 10 µM d'indométhacine (inhibiteur de prostaglandines synthétase) ont été ajoutés au milieu, pour réduire les contractions phasiques spontanées (Depoortere et al., Eur. J. Pharmacol. 515, 1-3, 160-168, 2003 ; Dass et al., Neurosciences 120, 443-453, 2003). Des contractions isotoniques ont été éveillées par stimulation par champ électrique. Deux électrodes de fil de platine ont été placées à la surface et au fond du bain d'organe et la stimulation par champ électrique a été réalisée par un stimulateur Power Lab (AD Instruments Pty Ltd. Castle Hill, Australie), couplé à un propulseur d'impulsion multiplex (Ugo Basile, Varese, Italie) (Fukuda et al., Scand. J. Gastroenterol. 12, 1209-1214, 2004). La stimulation supramaximale a été appliquée pour créer des contractions maximales (20 Hz, largeur de pulsation : 2 millisecondes ; 5 Volts ; trains de los toutes les 2 minutes, 150 mA). Ensuite, le courant a été réduit pour obtenir une stimulation submaximale (50% de réduction de la réponse contractile maximale). Les contractions ont été enregistrées par ordinateur avec un système d'enregistrement et d'analyse de données (Power Lab, Chart 5) relié à des transducteurs isotoniques (Ugo Basile, Varese, Italie) via des préamplificateurs (Octal Bridge Amp). Après stabilisation, des courbes cumulatives concentration-réponse de la ghréline (0,1 nM-1 µM) ont été tracées, avec et sans incubation (temps de contact : 30 mn) des molécules antagonistes. La stimulation par champ électrique supramaximale a été utilisée pour chaque bande comme référence (100 %) pour classer les réponses par substance test. La concentration agoniste produisant 50% d'effet maximal (EC50) a été calculée en utilisant un modèle logistique à quatre paramètres selon Ratkovsky et Reedy (Biometrics, 42, 575-582, 1986), avec ajustement par régression non linéaire en utilisant l'algorithme de Levenberg-Marquard dans le logiciel Everstat. Les valeurs pKB des antagonistes ont été calculées selon l'équation de Cheng-Prusoff (Kenakin et al., Competitive Antagonism, Pharmacologic Analysis of Drug-Receptor Interaction, 3e edition, 331-373, Philadelphie, New York ; Raven: Lippincott, 1997).

Les composés de formule (I) présentent une activité antagoniste du récepteur de la ghréline avec des CI₅₀ qui varient de 10⁻⁶ à 10⁻¹¹M.
Par exemple, les composés n° 1 et 2 ont montré une CI₅₀ de respectivement 5 10⁻⁸ M et 1 10⁻⁹M

Il apparaît donc que les composés selon l'invention ont une activité antagoniste du récepteur de la ghréline.

Les composés selon l'invention peuvent donc être utilisés pour la préparation de médicaments, en particulier de médicaments destinés à prévenir ou à traiter toute pathologie où le récepteur de la ghréline est impliqué.
Ainsi, selon un autre de ses aspects, l'invention a pour objet des médicaments qui comprennent un composé de formule (I) ou un sel d'addition de ce dernier à un acide pharmaceutiquement acceptable.
Ainsi, les composés selon l'invention peuvent être utilisés, chez l'homme ou chez l'animal, dans le traitement ou la prévention de différentes affections ghréline dépendantes. Ainsi, les composés selon l'invention peuvent être utilisés comme agents anorexiques, pour réguler l'appétit, la prise de repas et leur fréquence, ainsi que, à long terme, le poids, notamment la prise de poids faisant suite aux régimes diététiques ou thérapeutiques. Les composés selon l'invention sont donc particulièrement utiles pour la prévention ou le traitement de l'obésité, des troubles de l'appétit, du diabète, la surcharge pondérale et/ou de leurs effets.
Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, ainsi qu'au moins un excipient pharmaceutiquement acceptable.
Les dits.excipients sont choisis selon la forme pharmaceutique et le mode d'administration souhaité, parmi les excipients habituels qui sont connus de l'Homme du métier.
Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains, pour la prophylaxie ou le traitement des troubles ou des maladies ci-dessus.
Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 0,1 à 100 mg/kg, en une ou plusieurs prises. Par voie parentérale, elle peut atteindre 0.01 à 10 mg/Kg/jour.
Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

### Associations possibles

La présente invention concerne également les combinaisons d'un ou plusieurs composés(s) selon l'invention de formule générale (I) avec un ou plusieurs ingrédient(s) actif(s).

A titre d'ingrédient(s) actif(s) convenant auxdites combinaisons, on peut notamment citer les agents anti-obésité et antidiabétiques, ainsi que le rimonabant, la metformine ou les sulfonylurées.

La présente invention, selon un autre de ses aspects, concerne également un composé de formule (I), ou un de ses sels pharmaceutiquement acceptables, pour le traitement des pathologies ci-dessus indiquées.

## Revendications

1. Composé répondant à la formule (I) : 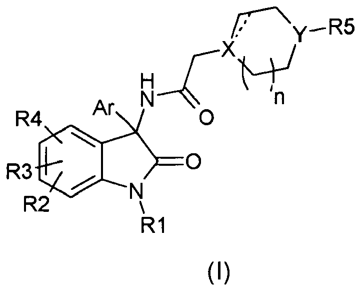 dans laquelle : 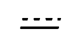
représente une simple ou double liaison,
X représente -N<, -CH< ou 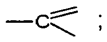
Y représente >N- ou >CH-, étant entendu qu'au moins un des X, Y représente N ;
Ar représente un groupe aryle ou hétéroaryle éventuellement substitué par un plusieurs substituants, identiques ou différents, choisis parmi les atomes d'halogène, les groupes (C1-6) alkyle, (C1-6) halogénoalkyle, perhalogéno(C1-3) alkyle, (C1-6) alcoxy, perhalogéno(C1-3) alcoxy, aryle;
R1 représente un atome d'hydrogène ou un groupe (C1-6) alkyle, - C(=O)(C1-6) alkyle, -C(=O)aryle;
R2, R3, R4, identiques ou différents, situés sur l'une quelconque des positions disponibles du noyau phényle, représentent indépendamment un atome d'hydrogène, un atome d'halogène, CN , OH, un groupe (C1-6) alkyle éventuellement substitué par un atome d'halogène ou un OH ; perhalogéno(C1-3) alkyle, (C1-6) alcoxy, perhalogéno(C1-3) alcoxy , aminocarbonyle, (C1-6) alkylaminocarbonyle, di(C1-6) alkylaminocarbonyle, aryle, aryloxy ; hétéroaryle ; le groupe aryle, aryloxy ou hétéroaryle pouvant éventuellement être substitué par un atome d'halogène, CN, OH ou un groupe (C1-6) alkyle , perhalogéno(C1-3) alkyle ou (C1-6) alcoxy ; étant entendu que au moins un des R2, R3, R4 est différent de H et que le groupe aryle, aryloxy ou hétéroaryle peut éventuellement être substitué par un atome d'halogène, CN, OH ou un groupe (C1-6) alkyle , perhalogéno(C1-3) alkyle, (C1-6) alcoxy;
R5 représente un groupe (C1-6) alkyle ou un (C2-6) alcényle; et
n représente 1 ou 2 ;
sous forme de base ou de sel d'addition à un acide ; à l'exclusion du 5-chloro-3-(2-chlorophényl)-1,3-dihydro-3-[2-(4-méthylpiperazin-1-yl)acétamido]indol-2-one .

2. Composé selon la revendication 1 tel que dans la formule générale (I) : 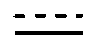
représente une simple ou double liaison ;
X représente -CH<, -N< ou 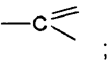
Y représente >N- ou >CH- ; étant entendu qu'au moins un des X, Y représente N ;
Ar représente un groupe aryle ou hétéroaryle éventuellement substitué par un plusieurs substituants, identiques ou différents, choisis parmi les atomes d'halogène, les groupes (C1-6) alcoxy, aryle, perhalogéno(C1-3) alkyle, (C1-6) alkyle ;
R1 représente un atome d'hydrogène ou un groupe -C(=O)(C1-6) alkyle, - C(=O)aryle, (C1-6) alkyle ;
R2, R3, R4, identiques ou différents, situés sur l'une quelconque des positions disponibles du noyau phényle, .représentent indépendamment un atome d'hydrogène, un atome d'halogène ou un groupe (C1-6) alkyle, perhalogéno(C1-3) alkyle, CN, aryle, hétéroaryle, OH, (C1-6) alcoxy, perhalogéno(C1-3) alcoxy , aminocarbonyle, (C1-6) alkylaminocarbonyle, di(C1-6) alkylaminocarbonyle, étant entendu que au moins un des R2, R3, R4 est différent de H ;
R5 représente un groupe (C1-6) alkyle ;
n représente 1 ou 2 ;
sous forme de base ou de sel d'addition à un acide.

3. Composé selon la revendication 1 ou 2 tel que dans la formule générale (I) : 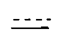
représente une simple ou double liaison ;
X représente -N<, -CH< ou 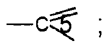
Y représente >N- ou >CH- ;
étant entendu qu'au moins un des X, Y représente N ;
Ar représente un groupe aryle éventuellement substitué par un ou plusieurs substituants choisis parmi les atomes d'halogène, et les groupes (C1-6) alcoxy, (C1-6) alkyle, aryle, trifluorométhyle , trifluorométhoxy ;
R1 représente un atome d'hydrogène ou un groupe -C(=O)(C1-6) alkyle, -C(=O)aryle, (C1-6) alkyle ;
R2, R3, R4, identiques ou différents, situés sur l'une quelconque des positions disponibles du noyau phényle, représentent indépendamment un atome d'hydrogène, un atome d'halogène, ou un groupe (C1-6) alkyle ou trifluorométhyle, étant entendu que au moins un des R2, R3, R4 est différent de H ;
R5 représente un groupe (C1-6) alkyle;
n représente 1 ou 2 ;
sous forme de base ou de sel d'addition à un acide.

4. Composé selon l'une quelconque des revendications précédentes tel que dans la formule générale (I) : 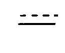
représente une simple ou double liaison ;
X représente -N<, -CH< ou 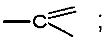
Y représente >N- ou >CH- ; étant entendu qu'au moins un des X ou Y représente N ;
Ar représente un groupe phényle ou naphtyle éventuellement substitué par un ou plusieurs substituants choisis parmi les atomes d'halogène, et les groupes méthoxy, méthyle, tert-butyle, phényle, trifluorométhyle , trifluorométhoxy;
R1 représente un atome d'hydrogène ou un groupe - C(=O)méthyle, -C(=O)phényle, méthyle ;
R2, R3, R4, identiques ou différents, situés sur l'une quelconque des positions disponibles du noyau phényle, représentent indépendamment un atome d'hydrogène, un atome d'halogène, ou un groupe trifluorométhyle, étant entendu que au moins un des R2, R3, R4 est différent de H ;
R5 représente un groupe méthyle, éthyle ou 2-propyle;
n représente 1 ou 2 ;
sous forme de base ou de sel d'addition à un acide.

5. Composé selon la revendication 1 ou 2 tel que dans la formule générale (I) :
Ar représente un groupe hétéroaryle éventuellement substitué par un ou plusieurs substituants, identiques ou différents, choisis parmi les atomes d'halogène, les groupes (C1-6) alcoxy, aryle, perhalogéno(C1-3) alkyle, (C1-6) alkyle.

6. Composé selon l'une quelconque des revendications précédentes choisi parmi les composés suivants :
(+) N-[5,6-Dichloro-3-(4-chloro-phényl)-2-oxo-2,3-dihydro-1H-indol-3-yl]-2-(4-méthyl-pipérazin-1-yl)-acétam ide
(+) N-[4,6-Dichloro-3-(4-trifluorométhyl-phényl)-2-oxo-2,3-dihydro-1H-indol-3-yl]-2-(1-ethyl-piperidin-4-yl)-acétamide
N-[4,6-Dichloro-3-(4-chloro-phényl)-2-oxo-2,3-dihydro-1H-indol-3-yl]-2-(4-méthyl-pipérazin-1-yl)-acétamide
N-[4-trifluoromethyl-6-cyano-3-(4-chloro-phényl)-2-oxo-2,3-dihydro-1H-indol-3-yl]-2-(4-éthyl-pipérazin-1-yl)-acétamide
(+) N-[1-Benzoyl-5,6-Dichloro-3-(4-chloro-phényl)-2-oxo-2,3-dihydro-1H-indol-3-yl]-2-(4-méthyl-pipérazin-1-yl)-acétamide
3-(4-Chloro-phényl)-3-[2-(4-ethyl-pipérazin-1-yl)-acétylamino]-2-oxo-4-trifluorométhyl-2,3-dihydro-1*H*-indole-6-carboxamide
*N*-[6-Chloro-3-(4-chloro-phényl)-1,5-diméthyl-2-oxo-2,3-dihydro-1*H*-indol-3-yl]-2-(4-méthyl-pipérazin-1-yl)-acétam ide
(+) N-[4,6-Dichloro-3-(4-trifluorométhyl-phényl)-2-oxo-2,3-dihydro-1H-indol-3-yl]-2-(1-ethyl-1,2,3,6-tetrahydro-pyrid-4-yl)-acétamide
N-[4,6-Dichloro-3-(3,4-dichloro-phényl)-2-oxo-2,3-dihydro-1H-indol-3-yl]-2-(4-éthyl-pipérazin-1-yl)-acétam ide
N-[4,6-Dichloro-3-(3-fluoro-4-chloro-phényl)-2-oxo-2,3-dihydro-1H-indol-3-yl]-2-(4-éthyl-pipérazin-1-yl)-acétam ide
N-[4,6-Dichloro-3-(3-trifluorométhyl-4-chloro-phényl)-2-oxo-2,3-dihydro-1H-indol-3-yl]-2-(4-éthyl-pipérazin-1-yl)-acétamide
N-[4,6-Dichloro-1-ethyl-3-(2-methyl-benzo[b]thiophen-5-yl)-2-oxo-2,3-dihydro-1H-indol-3-yl]-2-(1-ethyl-piperidin-4-yl)-acétamide
N-[4,6-Dichloro-1-ethyl-3-(2-methyl-benzofuran-5-yl)-2-oxo-2,3-dihydro -1 H-indol-3-yl]-2-(4-ethyl-piperazin-1-yl)-acetamide
N-[4,6-Dichloro-3-(4-trifluorométhoxy-phényl)-2-oxo-2,3-dihydro-1H-indol-3-yl]-2-(4-éthyl-pipérazin-1-yl)-acétamide
sous forme de base ou de sel d'addition à un acide.

7. Procédé de préparation d'un composé de formule (I) selon l'une quelconque des revendications 1 à 6, **caractérisé en ce qu'**il comprend une étape consistant à faire réagir un composé de formule générale (V) : 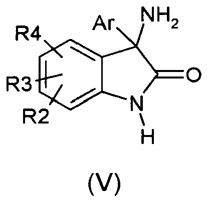 dans laquelle R2, R3, R4, Ar sont définis selon l'une quelconque des revendications 1 à 6.

8. Procédé selon la revendication 7 comprenant les étapes consistant à :
- faire réagir ledit composé de formule générale (V) avec un composé de formule générale (VI) : 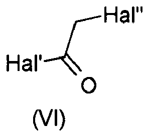 dans laquelle Hal' et Hal", identiques ou différents représentent indépendamment un atome d'halogène ;
- puis faire réagir le composé de formule générale (III) obtenu 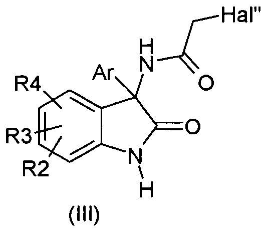 avec un composé de formule générale (IV) : 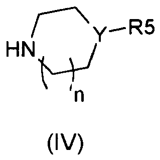 dans lesquelles R2, R3, R4, R5, Y, Ar, n sont tels que définis en formule générale (I), et Hal" représente un atome d'halogène ;
- suivie éventuellement de l'étape consistant à faire réagir le produit de formule (I) obtenu dans laquelle X représente -N< et R1 est égal à H, avec un composé de formule (II) :
R1-Hal (II)
dans laquelle R1, différent de H est défini comme en formule générale (I) et Hal représente un atome d'halogène.

9. Procédé selon la revendication 7 comprenant l'étape consistant à faire réagir ledit composé de formule générale (V) avec un composé de formule générale (VIII) : 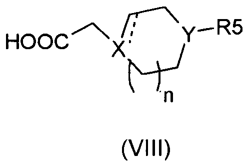 dans laquelle 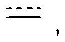 X, Y, R5 et n sont définis selon l'une quelconque des revendications 1 à 6,
suivie éventuellement de l'étape consistant à faire réagir le produit de formule (I) obtenu dans laquelle R1 est égal à H, avec un composé de formule (II) :
R1-Hal (II)
dans laquelle R1, différent de H est défini comme en formule générale (I) et Hal représente un atome d'halogène.

10. Procédé de préparation d'un composé de formule (I) selon l'une quelconque des revendications 1 à 6, **caractérisé en ce qu'**il comprend une étape consistant à faire réagir un composé de formule générale (XXVIII) : 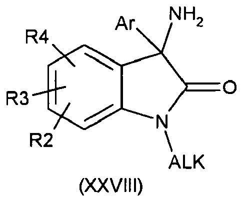 dans laquelle R2, R3, R4, Ar sont définis selon l'une quelconque des revendications 1 à 6 et ALK représente un groupe alkyle.

11. Procédé selon la revendication 10 comprenant les étapes consistant à :
- faire réagir ledit composé de formule générale (XXVIII) avec un composé de formule générale (VI) : 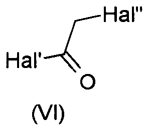 dans laquelle Hal' et Hal", identiques ou différents représentent indépendamment un atome d'halogène ;
- puis faire réagir le composé de formule générale (XXIX) obtenu 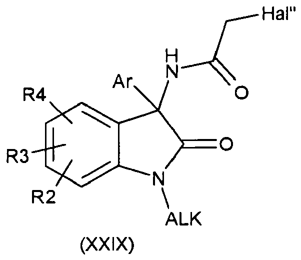 avec un composé de formule générale (IV) : 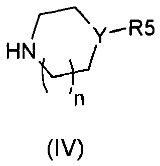 dans lesquelles R2, R3, R4, R5, Y, Ar, n sont tels que définis en formule générale (I), et Hal" représente un atome d'halogène.

12. Procédé selon la revendication 10 comprenant l'étape consistant à faire réagir ledit composé de formule générale (XXVIII) avec un composé de formule générale (VII) : 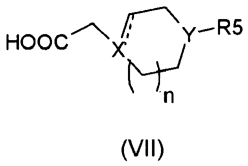 dans laquelle 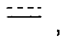 X, Y, R5 et n sont définis selon l'une quelconque des revendications 1 à 6.

13. Procédé selon l'une quelconque des revendications 7 à 12 comprenant l'étape ultérieure consistant à séparer le composé de formule générale (I) désiré.

14. Composé de formule (III) : 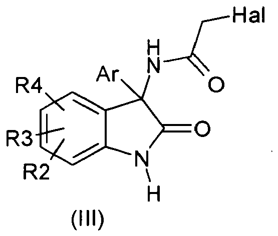 dans laquelle R2, R3, R4, Ar sont définis selon l'une quelconque des revendications 1 à 6 et Hal représente un atome d'halogène, à l'exclusion du 5-chloro-3-(2-chloroacétamido)-3-(2-chlorophényl)-1,3-dihydroindol-2-one.

15. Composé de formule (III) selon la revendication 14 dans laquelle :
Ar représente un groupe hétéroaryle éventuellement substitué par un ou plusieurs substituants, identiques ou différents, choisis parmi les atomes d'halogène, les groupes (C1-6) alcoxy, aryle, perhalogéno(C1-3) alkyle, (C1-6) alkyle.

16. Composé de formule générale (XXVIII) : 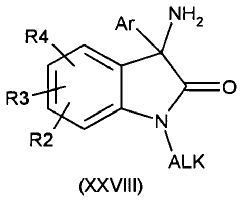 dans laquelle R2, R3, R4, Ar sont définis selon l'une quelconque des revendications 1 à 6 et ALK représente un groupe alkyle.

17. Composé de formule générale (XXIX) : 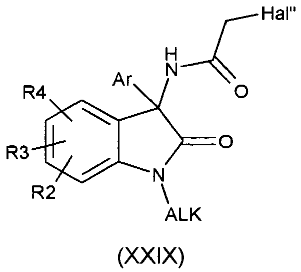 dans laquelle R2, R3, R4, Ar sont définis selon l'une quelconque des revendications 1 à 6, ALK représente un groupe alkyle et Hal" représente un atome d'halogène.

18. Médicament, **caractérisé en ce qu'**il comprend un composé de formule (I) selon l'une quelconque des revendications 1 à 6, ou un sel d'addition de ce composé à un acide pharmaceutiquement acceptable.

19. Composition pharmaceutique, **caractérisée en ce qu'**elle comprend un composé de formule (I) selon l'une quelconque des revendications 1 à 6, ou un sel pharmaceutiquement acceptable.

20. Utilisation d'un composé selon l'une quelconque des revendications 1 à 4 pour la préparation d'un médicament destiné à la prévention ou au traitement de l'obésité, le diabète, les troubles de l'appétit et la surcharge pondérale.

21. Composé selon l'une quelconque des revendications 1 à 6 pour la prévention ou le traitement de l'obésité, le diabète, les troubles de l'appétit et la surcharge pondérale.

22. Combinaison comprenant un ou plusieurs composés(s) selon l'une quelconque des revendications 1 à 6 avec un ou plusieurs ingrédient(s) actif(s).

## Claims

1. Compound corresponding to formula (I): 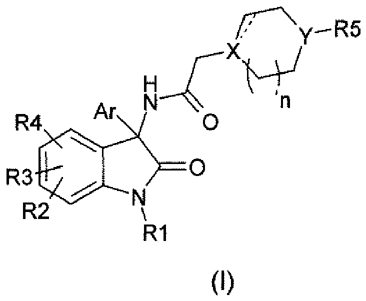 in which: 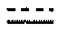
represents a single or double bond,
X represents -N<, -CH< or 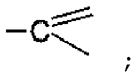
Y represents >N- or >CH-, it being understood that at least one from among X and Y represents N;
Ar represents an aryl or heteroaryl group optionally substituted with one or more substituents, which may be identical or different, chosen from halogen atoms and (C1-6)alkyl, (C1-6)haloalkyl, perhalo(C1-3)alkyl, (C1-6)alkoxy, perhalo(C1-3)alkoxy and aryl groups;
R1 represents a hydrogen atom or a (C1-6)alkyl, - C(=O)(C1-6)alkyl or -C(=O)aryl group;
R2, R3 and R4, which may be identical or different, located on any of the available positions of the phenyl nucleus, independently represent a hydrogen atom, a halogen atom, CN, OH, a (C1-6)alkyl group optionally substituted with a halogen atom or an OH; perhalo(C1-3)alkyl, (C1-6)alkoxy, perhalo(C1-3)alkoxy, aminocarbonyl, (C1-6)alkylaminocarbonyl, di (C1-6)alkylaminocarbonyl, aryl, aryloxy; heteroaryl; the aryl, aryloxy or heteroaryl group possibly being optionally substituted with a halogen atom, CN, OH or a (C1-6) alkyl, perhalo (1-3) alkyl or (C1-6) alkoxy group; it being understood that at least one from among R2, R3 and R4 is other than H and that the aryl, aryloxy or heteroaryl group may be optionally substituted with a halogen atom, CN, OH or a (C1-6)alkyl, perhalo(C1-3)alkyl or (C1-6)alkoxy group;
R5 represents a (C1-6)alkyl or (C2-6)alkenyl group; and
n represents 1 or 2;
in the form of the base or of an acid-addition salt; with the exclusion of 5-chloro-3-(2-chlorophenyl)-1,3-dihydro-3-[2-(4-methylpiperazin-1-yl)acetamido]indol-2-one.

2. Compound according to Claim 1, such that, in the general formula (I): 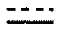
represents a single or double bond;
X represents -CH<, -N< or 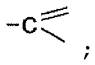
Y represents >N- or >CH-, it being understood that at least one from among X and Y represents N;
Ar represents an aryl or heteroaryl group optionally substituted with one or more substituents, which may be identical or different, chosen from halogen atoms and (C1-6)alkyl, perhalo(C1-3)alkyl, (C1-6)alkoxy and aryl groups;
R1 represents a hydrogen atom or a -C(=O)(C1-6)alkyl, -C(=O)aryl or (C1-6) alkyl group;
R2, R3 and R4, which may be identical or different, located on any of the available positions of the phenyl nucleus, independently represent a hydrogen atom, a halogen atom, a (C1-6)alkyl, perhalo(C1-3)alkyl, CN, aryl, heteroaryl, OH, (C1-6)alkoxy, perhalo(C1-3)alkoxy, aminocarbonyl, (C1-6)alkylaminocarbonyl or di(C1-6)alkylaminocarbonyl group, it being understood that at least one from among R2, R3 and R4 is other than H;
R5 represents a (C1-6)alkyl group;
n represents 1 or 2;
in the form of the base or of an acid-addition salt.

3. Compound according to Claim 1 or 2, such that, in the general formula (I): 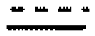
represents a single or double bond;
X represents -N<, -CH< or 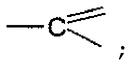
Y represents >N- or >CH-;
it being understood that at least one from among X and Y represents N;
Ar represents an aryl group optionally substituted with one or more substituents chosen from halogen atoms, and (C1-6)alkoxy, (C1-6)alkyl, aryl, trifluoromethyl and trifluoromethoxy groups;
R1 represents a hydrogen atom or a -C(=O)(C1-6)alkyl, -C(=O)aryl or (C1-6)alkyl group;
R2, R3 and R4, which may be identical or different, located on any of the available positions of the phenyl nucleus, independently represent a hydrogen atom, a halogen atom, or a (C1-6)alkyl or trifluoromethyl group, it being understood that at least one from among R2, R3 and R4 is other than H;
R5 represents a (C1-6)alkyl group;
n represents 1 or 2;
in the form of the base or of an acid-addition salt.

4. Compound according to any one of the preceding claims, such that, in the general formula (I): 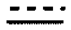
represents a single or double bond;
X represents -N<, -CH< or 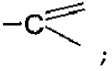
Y represents >N- or >CH-; it being understood that at least one from among X and Y represents N;
Ar represents a phenyl or naphthyl group optionally substituted with one or more substituents chosen from halogen atoms, and methoxy, methyl, tert-butyl, phenyl, trifluoromethyl and trifluoromethoxy groups;
R1 represents a hydrogen atom or a -C(=O)methyl, -C(=O)phenyl or methyl group;
R2, R3 and R4, which may be identical or different, located on any of the available positions of the phenyl nucleus, independently represent a hydrogen atom, a halogen atom or trifluoromethyl group, it being understood that at least one from among R2, R3 and R4 is other than H;
R5 represents a methyl, ethyl or 2-propyl group;
n represents 1 or 2;
in the form of the base or of an acid-addition salt.

5. Compound according to Claim 1 or 2, such that, in the general formula (I):
Ar represents a heteroaryl group optionally substituted with one or more substituents, which may be identical or different, chosen from halogen atoms and (C1-6)alkoxy, aryl, perhalo(C1-3)alkyl and (C1-6)alkyl groups.

6. Compound according to any one of the preceding claims, chosen from the following compounds:
(+)-N-[5,6-dichloro-3-(4-chlorophenyl)-2-oxo-2,3-dihydro-1H-indol-3-yl]-2-(4-methylpiperazin-1-yl)acetamide
(+)-N-[4,6-dichloro-3-(4-trifluoromethylphenyl)-2-oxo-2,3-dihydro-1H-indol-3-yl]-2-(1-ethylpiperid-4-yl)acetamide
N-[4,6-dichloro-3-(4-chlorophenyl)-2-oxo-2,3-dihydro-1H-indol-3-yl]-2-(4-methylpiperazin-1-yl)acetamide
N-[4-trifluoromethyl-6-cyano-3-(4-chlorophenyl)-2-oxo-2,3-dihydro-1H-indol-3-yl]-2-(4-ethylpiperazin-1-yl)acetamide
(+)-N-[1-benzoyl-5,6-dichloro-3-(4-chlorophenyl)-2-oxo-2,3-dihydro-1H-indol-3-yl]-2-(4-methylpiperazin-1-yl)acetamide
3-(4-chlorophenyl)-3-[2-(4-ethylpiperazin-1-yl)acetylamino]-2-oxo-4-trifluoromethyl-2,3-dihydro-1H-indole-6-carboxamide
N-[6-chloro-3-(4-chlorophenyl)-1,5-dimethyl-2-oxo-2,3-dihydro-1H-indol-3-yl]-2-(4-methylpiperazin-1-yl)acetamide
(+)-N-[4,6-dichloro-3-(4-trifluoromethylphenyl)-2-oxo-2,3-dihydro-1H-indol-3-yl]-2-(1-ethyl-1,2,3,6-tetrahydropyrid-4-yl)acetamide
N-[4,6-dichloro-3-(3,4-dichlorophenyl)-2-oxo-2,3-dihydro-1H-indol-3-yl]-2-(4-ethylpiperazin-1-yl)acetamide
N-[4,6-dichloro-3-(3-fluoro-4-chlorophenyl)-2-oxo-2,3-dihydro-1H-indol-3-yl]-2-(4-ethylpiperazin-1-yl)acetamide
N-[4,6-dichloro-3-(3-trifluoromethyl-4-chlorophenyl)-2-oxo-2,3-dihydro-1H-indol-3-yl]-2-(4-ethylpiperazin-1-yl)acetamide
N-[4,6-dichloro-1-ethyl-3-(2-methylbenzo[b]thiophen-5-yl)-2-oxo-2,3-dihydro-1H-indol-3-yl]-2-(1-ethylpiperid-4-yl)acetamide
N-[4,6-dichloro-1-ethyl-3-(2-methyl-5-benzofuryl)-2-oxo-2,3-dihydro-1H-indol-3-yl]-2-(4-ethylpiperazin-1-yl)acetamide
N-[4,6-dichloro-3-(4-trifluoromethoxyphenyl)-2-oxo-2,3-dihydro-1H-indol-3-yl]-2-(4-ethylpiperazin-1-yl)acetamide in the form of the base or of an acid-addition salt.

7. Process for preparing a compound of formula (I) according to any one of Claims 1 to 6, **characterized in that** it comprises a step that consists in reacting a compound of general formula (V): 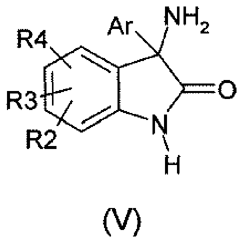 in which R2, R3, R4 and Ar are as defined according to any one of Claims 1 to 6.

8. Process according to Claim 7, comprising the steps consisting in:
- reacting the said compound of general formula (V) with a compound of general formula (VI): 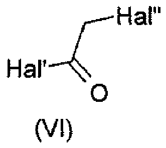 in which Hal' and Hal", which may be identical or different, independently represent a halogen atom;
- and then in reacting the compound of general formula (III) obtained 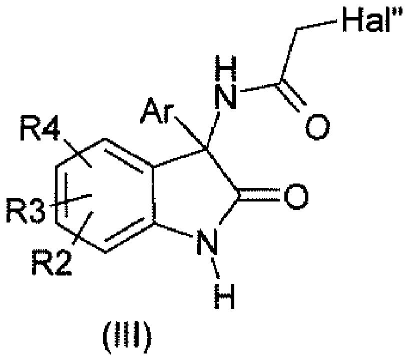 with a compound of general formula (IV): 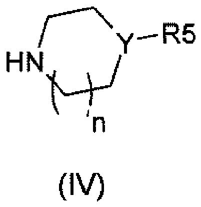 in which R2, R3, R4, R5, Y, Ar and n are defined as in the general formula (I) and Hal" represents a halogen atom;
- optionally followed by the step that consists in reacting the product of formula (I) obtained, in which X represents -N< and R1 is equal to H, with a compound of formula (II):
R1-Hal (II)
in which R1, which is other than H, is defined as in the general formula (I) and Hal represents a halogen atom.

9. Process according to Claim 7, comprising the step that consists in reacting the said compound of general formula (V) with a compound of general formula (VIII): 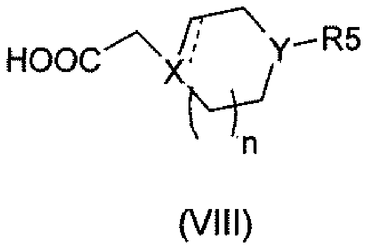 in which 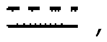 X, Y, R5 and n are as defined according to any one of Claims 1 to 6;
optionally followed by the step that consists in reacting the product of formula (I) obtained, in which R1 is equal to H, with a compound of formula (II):
R1-Hal (II)
in which R1, which is other than H, is defined as in the general formula (I) and Hal represents a halogen atom.

10. Process for preparing a compound of formula (I) according to any one of Claims 1 to 6, **characterized in that** it comprises a step that consists in reacting a compound of general formula (XXVIII): 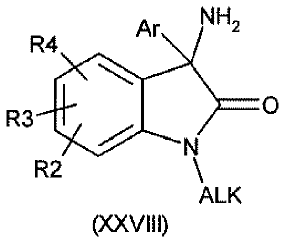 in which R2, R3, R4 and Ar are defined according to any one of Claims 1 to 6 and ALK represents an alkyl group.

11. Process according to Claim 10, comprising the steps that consist in
- reacting the said compound of general formula (XXVIII) with a compound of general formula (VI): 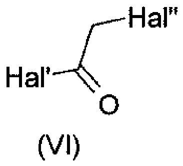 in which Hal' and Hal", which may be identical or different, independently represent a halogen atom;
- and then in reacting the compound of general formula (XXIX) obtained 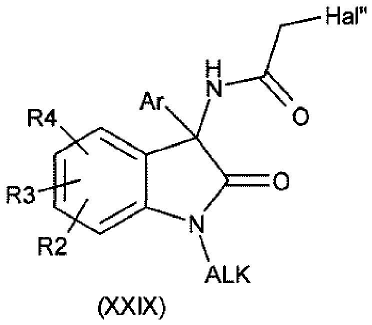 with a compound of general formula (IV): 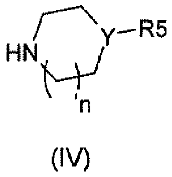 in which R2, R3, R4, R5, Y, Ar and n are as defined in the general formula (I), and Hal" represents a halogen atom.

12. Process according to Claim 10, comprising the step that consists in reacting the said compound of general formula (XXVIII) with a compound of general formula (VII): 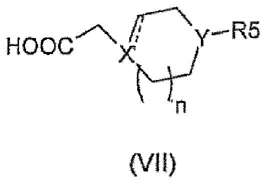 in which 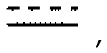 X, Y, R5 and n are defined according to any one of Claims 1 to 6.

13. Process according to any one of Claims 7 to 12, comprising the subsequent step that consists in separating out the desired compound of general formula (I) .

14. Compound of formula (III): 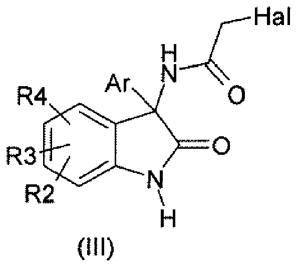 in which R2, R3, R4 and Ar are defined according to any one of Claims 1 to 6 and Hal represents a halogen atom, with the exclusion of 5-chloro-3-(2-chloroacetamido)-3-(2-chlorophenyl)-1,3-dihydroindol-2-one.

15. Compound of formula (III) according to Claim 14, in which:
Ar represents a heteroaryl group optionally substituted with one or more substituents, which may be identical or different, chosen from halogen atoms and (C1-6)alkoxy, aryl, perhalo(C1-3)alkyl and (C1-6)alkyl groups.

16. Compound of general formula (XXVIII): 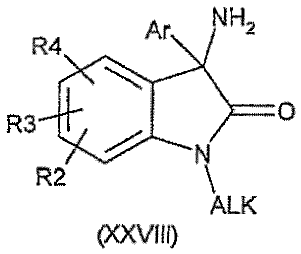 in which R2, R3, R4 and Ar are defined according to any one of Claims 1 to 6 and ALK represents an alkyl group.

17. Compound of general formula (XXIX): 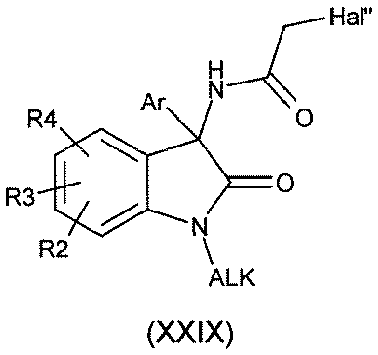 in which R2, R3, R4 and Ar are defined according to any one of Claims 1 to 6, ALK represents an alkyl group and Hal" represents a halogen atom.

18. Medicament, **characterized in that** it comprises a compound of formula (I) according to any one of Claims 1 to 6, or an addition salt of this compound with a pharmaceutically acceptable acid.

19. Pharmaceutical composition, **characterized in that** it comprises a compound of formula (I) according to any one of Claims 1 to 6, or a pharmaceutically acceptable salt.

20. Use of a compound according to any one of Claims 1 to 4 for the preparation of a medicament for preventing or treating obesity, diabetes, appetite disorders and excess weight.

21. Compound according to any one of Claims 1 to 6, for preventing or treating obesity, diabetes, appetite disorders and excess weight.

22. Combination comprising one or more compounds according to any one of Claims 1 to 6 with one or more active ingredient(s).

## Patentansprüche

1. Verbindung der Formel (I): 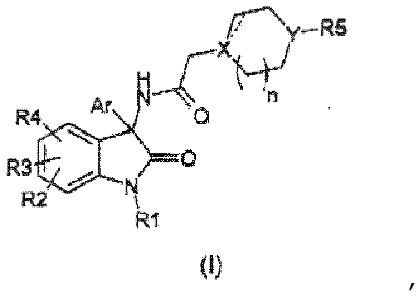 worin: 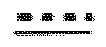
für eine Einfach- oder Doppelbindung steht,
X für -N<, -CH< oder 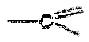 steht,
Y für >N- oder >CH- steht, wobei selbstverständlich ist, dass mindestens einer der Reste X, Y für N steht;
Ar für eine Aryl- oder Heteroarylgruppe steht, die gegebenenfalls mit einem oder mehreren Substituenten substituiert ist, die gleich oder verschieden sind und aus Halogenatomen, (C1-6)Alkyl-, (C1-6)Halogenalkyl-, Perhalogen(C1-3)alkyl-, (C1-6)Alkoxy-, Perhalogen(C1-3)alkoxy-, Arylgruppen ausgewählt sind;
R1 für ein Wasserstoffatom oder eine (C1-6)Alkyl-, -C(=O)(C1-6)Alkyl- oder -C(=O)Arylgruppe steht;
R2, R3, R4, die gleich oder verschieden sind und sich an einer beliebigen der verfügbaren Positionen des Phenylkerns befinden, unabhängig für ein Wasserstoffatom, ein Halogenatom, CN, OH, eine (C1-6)Alkylgruppe, die gegebenenfalls mit einem Halogenatom oder OH substituiert ist; Perhalogen(C1-3)alkyl, (C1-6)Alkoxy, Perhalogen(C1-3)alkoxy, Aminocarbonyl, (C1-6)Alkylaminocarbonyl, Di(C1-6)alkylaminocarbonyl, Aryl, Aryloxy, Heteroaryl stehen; wobei die Aryl-, Aryloxy-oder Heteroarylgruppe gegebenenfalls mit einem Halogenatom, CN, OH oder einer (C1-6)Alkyl-, Perhalogen(C1-3)alkyl- oder (C1-6)Alkoxygruppe substituiert sein kann; wobei selbstverständlich ist, dass mindestens einer der Reste R2, R3, R4 verschieden von H ist und dass die Aryl-, Aryloxy- oder Heteroarylgruppe gegebenenfalls durch ein Halogenatom, CN, OH oder eine (C1-C6)Alkyl-, Perhalogen(C1-3)alkyl- oder (C1-6)Alkoxygruppe substituiert sein kann;
R5 für eine (C1-6)Alkyl- oder (C2-6)Alkenylgruppe steht; und
n für 1 oder 2 steht;
in Form der Base oder eines Säureadditionssalzes;
unter Ausschluss von 5-Chlor-3-(2-chlorphenyl)-1,3-dihydro-3-[2-(4-methylpiperazin-1-yl)acetamido]indol-2-on.

2. Verbindung nach Anspruch 1, wie in der allgemeinen Formel (I):
worin 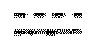 für eine Einfach- oder Doppelbindung steht,
X für -CH<, -N< oder 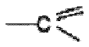 steht
Y für >N- oder >CH- steht, wobei selbstverständlich ist, dass mindestens einer der Reste X, Y für N steht;
Ar für eine Aryl- oder Heteroarylgruppe steht, die gegebenenfalls mit einem oder mehreren Substituenten substituiert ist, die gleich oder verschieden sind und aus Halogenatomen, (C1-6)Alkoxy-, Aryl-, Perhalogen(C1-3)alkyl-, (C1-6)Alkylgruppen ausgewählt sind;
R1 für ein Wasserstoffatom oder eine -C(=O)(C1-6)Alkyl-, -C(=O)Aryl-, (C1-6)Alkylgruppe steht;
R2, R3, R4, die gleich oder verschieden sind und sich an einer beliebigen der verfügbaren Positionen des Phenylkerns befinden, unabhängig für ein Wasserstoffatom, ein Halogenatom oder eine (C1-6)Alkyl-, Perhalogen(C1-3)alkyl-, CN, Aryl-, Heteroaryl-, OH, (C1-6)Alkoxy, Perhalogen(C1-3)alkoxy-, Aminocarbonyl-, (C1-6) Alkylaminocarbonyl-, Di(C1-6)alkylamino-carbonylgruppe stehen; wobei selbstverständlich ist, dass mindestens einer der Reste R2, R3, R4 verschieden von H ist;
R5 für eine (C1-6)Alkylgruppe steht;
n für 1 oder 2 steht;
in Form der Base oder eines Säureadditionssalzes.

3. Verbindung nach Anspruch 1 oder 2, wie in der allgemeinen Formel (I):
worin 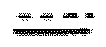 für eine Einfach- oder Doppelbindung steht,
X für -N<, -CH< oder 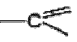 steht, Y für >N- oder >CH- steht,
wobei selbstverständlich ist, dass mindestens einer der Reste X, Y für N steht;
Ar für eine Arylgruppe steht, die gegebenenfalls mit einem oder mehreren Substituenten substituiert ist, die aus Halogenatomen und (C1-6)Alkoxy-,(C1-6)Alkyl-, Aryl-, Trifluormethyl-, Trifluormethoxy-gruppen ausgewählt sind;
R1 für ein Wasserstoffatom oder eine -C(=O)(C1-6)Alkyl-, -C(=O)Aryl-, (C1-6)Alkylgruppe steht;
R2, R3, R4, die gleich oder verschieden sind und sich an einer beliebigen der verfügbaren Positionen des Phenylkerns befinden, unabhängig für ein Wasserstoffatom, ein Halogenatom oder eine (C1-6)Alkyl- oder Trifluormethylgruppe stehen; wobei selbstverständlich ist, dass mindestens einer der Reste R2, R3, R4 verschieden von H ist;
R5 für eine (C1-6)Alkylgruppe steht;
n für 1 oder 2 steht;
in Form der Base oder eines Säureadditionssalzes.

4. Verbindung nach einem der vorhergehenden Ansprüche, wie in der allgemeinen Formel (I):
worin 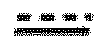 für eine Einfach- oder Doppelbindung steht,
X für -N<, -CH< oder 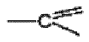 steht,
Y für >N- oder >CH- steht, wobei selbstverständlich ist, dass mindestens einer der Reste X, Y für N steht;
Ar für eine Phenyl- oder Naphthylgruppe steht, die gegebenenfalls mit einem oder mehreren Substituenten substituiert ist, die aus Halogenatomen und Methoxy-, Methyl-, tert.-Butyl-, Phenyl-, Trifluormethyl-, Trifluormethoxygruppen ausgewählt sind;
R1 für ein Wasserstoffatom oder eine -C(=0)Methyl-, -C(=0)Phenyl-, Methylgruppe steht;
R2, R3, R4, die gleich oder verschieden sind und sich an einer beliebigen der verfügbaren Positionen des Phenylkerns befinden, unabhängig für ein Wasserstoffatom, ein Halogenatom oder eine Trifluormethylgruppe stehen; wobei selbstverständlich ist, dass mindestens einer der Reste R2, R3, R4 verschieden von H ist;
R5 für eine Methyl-, Ethyl- oder 2-Propylgruppe steht;
n für 1 oder 2 steht;
in Form der Base oder eines Säureadditionssalzes.

5. Verbindung nach Anspruch 1 oder 2, wie in der allgemeinen Formel (I), worin:
Ar für eine Heteroarylgruppe steht, die gegebenenfalls mit einem oder mehreren Substituenten substituiert ist, die gleich oder verschieden sind und aus Halogenatomen, den (C1-6)Alkoxy-, Aryl-, Perhalogen(C1-3)alkyl-, (C1-6)Alkylgruppen ausgewählt sind.

6. Verbindung nach einem der vorhergehenden Ansprüche, die aus den folgenden Verbindungen ausgewählt wird:
(+)-N-[5,6-Dichlor-3-(4-chlorphenyl)-2-oxo-2,3-dihydro-1H-indol-3-yl]-2-(4-methylpiperazin-1-yl)acetamid
(+)-N-[4,6-Dichlor-3-(4-trifluormethylphenyl)-2-oxo-2,3-dihydro-1H-indol-3-yl]-2-(1-ethylpiperid-4-yl)acetamid
N-[4,6-Dichlor-3-(4-chlorphenyl)-2-oxo-2,3-dihydro-1H-indol-3-yl]-2-(4-methylpiperazin-1-yl)acetamid
N-[4-Trifluormethyl-6-cyano-3-(4-chlorphenyl)-2-oxo-2,3-dihydro-1H-indol-3-yl]-2-(4-ethylpiperazin-1-yl)acetamid
(+)-N-[1-Benzoyl-5,6-dichlor-3-(4-chlorphenyl)-2-oxo-2,3-dihydro-1H-indol-3-yl]-2-(4-methylpiperazin-1-yl)acetamid
3-(4-Chlorphenyl)-3-[2-(4-ethylpiperazin-1-yl)acetylamino]-2-oxo-4-trifluormethyl-2,3-dihydro-1H-indol-6-carboxamid
N-[6-Chlor-3-(4-chlorphenyl)-1,5-dimethyl-2-oxo-2,3-dihydro-1H-indol-3-yl]-2-(4-methylpiperazin-1-yl)acetamid
(+)-N-[4,6-Dichlor-3-(4-trifluormethylphenyl)-2-oxo-2,3-dihydro-1H-indol-3-yl]-2-(1-ethyl-1,2,3,6-tetrahydropyrid-4-yl)acetamid
N-[4,6-Dichlor-3-(3,4-dichlorphenyl)-2-oxo-2,3-dihydro-1H-indol-3-yl]-2-(4-ethylpiperazin-1-yl)acetamid
N-[4,6-Dichlor-3-(3-fluor-4-chlorphenyl)-2-oxo-2,3-dihydro-1H-indol-3-yl]-2-(4-ethylpiperazin-1-yl)acetamid
N-[4,6-Dichlor-3-(3-trifluormethyl-4-chlorphenyl)-2-oxo-2,3-dihydro-1H-indol-3-yl]-2-(4-ethylpiperazin-1-yl)acetamid
N-[4,6-Dichlor-1-ethyl-3-(2-methylbenzo[b]thiophen-5-yl)-2-oxo-2,3-dihydro-1H-indol-3-yl]-2-(1-ethylpiperid-4-yl)acetamid
N-[4,6-Dichlor-1-ethyl-3-(2-methylbenzofuran-5-yl)-2-oxo-2,3-dihydro-1H-indol-3-yl]-2-(4-ethylpiperazin-1-yl)acetamid
N-[4,6-Dichlor-3-(4-trifluormethoxyphenyl)-2-oxo-2,3-dihydro-1H-indol-3-yl]-2-(4-ethylpiperazin-1-yl)acetamid
in Form der Base oder eines Säureadditionssalzes.

7. Verfahren zur Herstellung einer Verbindung der Formel (I) nach einem der Ansprüche 1 bis 6, **dadurch gekennzeichnet, dass** man in einem Schritt eine Verbindung der allgemeinen Formel (V): 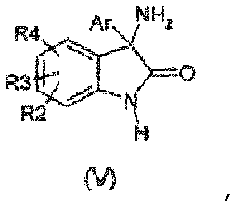 worin R2, R3, R4, Ar wie nach einem der Ansprüche 1 bis 6 definiert sind, umsetzt.

8. Verfahren nach Anspruch 7 mit den folgenden Schritten:
- Umsetzen der Verbindung der allgemeinen Formel (V) mit einer Verbindung der allgemeinen Formel (VI): 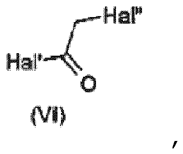 worin Hal' und Hal'', die gleich oder verschieden sind, unabhängig für ein Halogenatom stehen;
- anschließend Umsetzen der erhaltenen Verbindung der allgemeinen Formel (III) 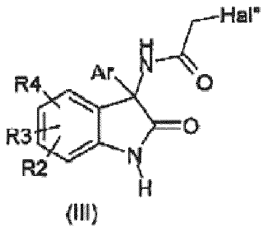 mit einer Verbindung der allgemeinen Formel (IV): 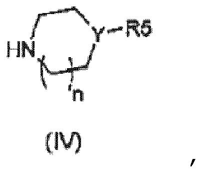 worin R2, R3, R4, R5, Y, Ar, n wie in der allgemeinen Formel (I) definiert sind und Hal'' für ein Halogenatom steht;
- und schließlich Umsetzen des erhaltenen Produkts der Formel (I), worin X für -N< steht und R1 gleich H ist, mit einer Verbindung der Formel (II):
R1-Hal (II),
worin R1 nicht H ist und wie in der allgemeinen Formel (I) definiert ist und Hal für ein Halogenatom steht.

9. Verfahren nach Anspruch 7, wobei man in einem Schritt die Verbindung der allgemeinen Formel (V) mit einer Verbindung der allgemeinen Formel (VIII): 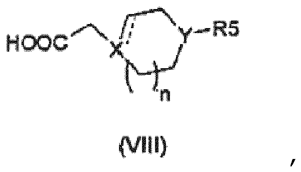 worin 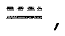 X, Y, R5 wie in einem der Ansprüche 1 bis 6 definiert sind, umsetzt
und schließlich das erhaltene Produkt der Formel (I), worin R1 gleich H ist, mit einer Verbindung der Formel (II):
R1-Hal (II),
worin R1 nicht H ist und wie in der allgemeinen Formel (I) definiert ist und Hal für ein Halogenatom steht, umsetzt.

10. Verfahren zur Herstellung einer Verbindung der Formel (I) nach einem der Ansprüche 1 bis 6, **dadurch gekennzeichnet, dass** man in einem Schritt eine Verbindung der allgemeinen Formel (XXVIII): 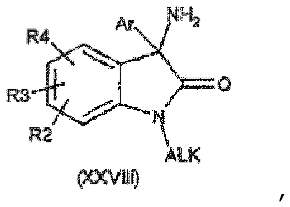 worin R2, R3, R4, Ar wie in einem der Ansprüche 1 bis 6 definiert sind und ALK für eine Alkylgruppe steht, umsetzt.

11. Verfahren nach Anspruch 10 mit den folgenden Schritten:
- Umsetzen der Verbindung der allgemeinen Formel (XXVIII) mit einer Verbindung der allgemeinen Formel (VI): 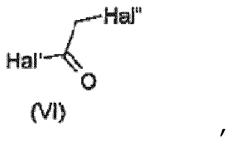 worin Hal' und Hal'', die gleich oder verschieden sind, unabhängig für ein Halogenatom stehen;
- anschließend Umsetzen der erhaltenen Verbindung der allgemeinen Formel (XXIX) 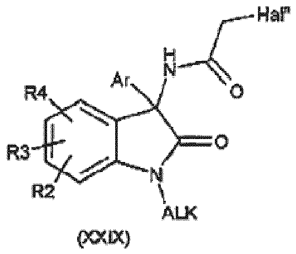 mit einer Verbindung der allgemeinen Formel (IV): 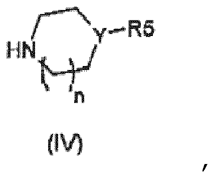 worin R2, R3, R4, R5, Y, Ar, n wie in der allgemeinen Formel (I) definiert sind und Hal'' für ein Halogenatom steht.

12. Verfahren nach Anspruch 10, wobei man in einem Schritt die Verbindung der allgemeinen Formel (XXVIII) mit einer Verbindung der allgemeinen Formel (VII): 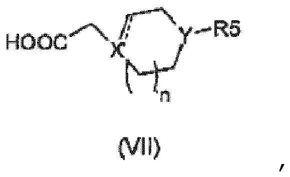 worin 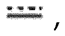 X, Y, R5 und n wie in einem der Ansprüche 1 bis 6 definiert sind, umsetzt.

13. Verfahren nach einem der Ansprüche 7 bis 12, wobei man in einem abschließenden Schritt die gewünschte Verbindung der allgemeinen Formel (I) abtrennt.

14. Verbindung der Formel (III): 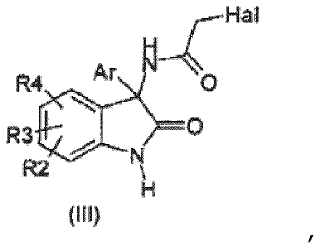 worin R2, R3, R4, Ar gemäß einem der Ansprüche 1 bis 6 definiert sind und Hal für ein Halogenatom steht, unter Ausschluss von von 5-Chlor-3-(2-chloracetamido)-3-(2-chlorphenyl)-1,3-dihydroindol-2-on.

15. Verbindung der Formel (III) nach Anspruch 14, worin:
Ar für eine Heteroarylgruppe steht, die gegebenenfalls mit einem oder mehreren Substituenten substituiert ist, die gleich oder verschieden sind und aus Halogenatomen, (C1-6)Alkoxy-, Aryl-, Perhalogen(C1-3)alkyl-, (C1-6)alkylgruppen ausgewählt werden.

16. Verbindung der allgemeinen Formel (XXVIII): 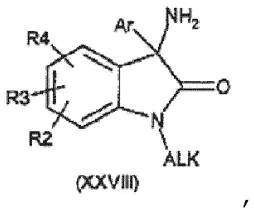 worin R2, R3, R4, Ar gemäß einem der Ansprüche 1 bis 6 definiert sind und ALK für eine Alkylgruppe steht.

17. Verbindung der allgemeinen Formel (XXIX): 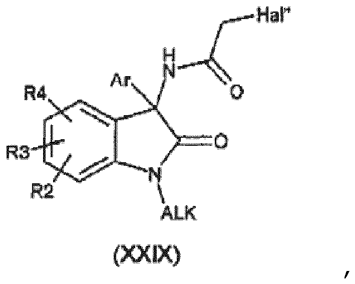 worin R2, R3, R4, Ar gemäß einem der Ansprüche 1 bis 6 definiert sind, ALK für eine Alkylgruppe steht und Hal'' für ein Halogenatom steht.

18. Arzneimittel, **dadurch gekennzeichnet, dass** es eine Verbindung der Formel (I) nach einem der Ansprüche 1 bis 6 oder ein Additionssalz dieser Verbindung mit einer pharmazeutisch annehmbaren Säure umfasst.

19. Pharmazeutische Zusammensetzung, **dadurch gekennzeichnet, dass** sie eine Verbindung der Formel (I) nach einem der Ansprüche 1 bis 6 oder ein pharmazeutisch annehmbares Salz umfasst.

20. Verwerdung einer Verbindung nach einem der Ansprüche 1 bis 4 zur Herstellung eines Arzneimittels für die Prävention oder Behandlung von Fettleibigkeit, Diabetes, Appetitstörungen und Übergewicht.

21. Verbindung nach einem der Ansprüche 1 bis 6 zur Prävention oder Behandlung von Fettleibigkeit, Diabetes, Appetitstörungen und Übergewicht.

22. Kombination, die eine oder mehrere Verbindung(en) nach einem der Ansprüche 1 bis 6 mit einem oder mehreren Wirkstoff(en) umfasst.
